# EUROPÄISCHE PATENTANMELDUNG

(11) **EP 4 397 323 A1**
(43) Veröffentlichungstag der Anmeldung: **10.07.2024**
(21) Anmeldenummer: 23150759.1
(22) Anmeldetag: 09.01.2023
(51) Int. Cl.: A61L 2/00

(54) **VERFAHREN ZUR INAKTIVIERUNG VON VIREN IN EINEM FLUID**

(71) Anmelder: Sartorius Stedim Biotech GmbH, 37079 Göttingen (DE)
(72) Erfinder: ZILAEV, ALexander, 37079 Göttingen (DE); LEUTHOLD, Martin, 37079 Göttingen (DE); GRÜNBERG, Mario, 37079 Göttingen (DE); WORTMEYER, Johannes, 37079 Göttingen (DE)
(74) Vertreter: Gottschald Patentanwälte Partnerschaft mbB

(57) **Zusammenfassung**

Die Erfindung betrifft ein Verfahren zur Inaktivierung von Viren in einem Fluid, bei welchem ein mit aktiven Viren belastetes Ausgangsfluid (AF) in einer ersten Vermischungsanordnung (2) mit einem vireninaktivierenden Reagenz (R1) zu einem reaktiven Fluid (F) vermischt wird und die aktiven Viren durch Inkubation des reaktiven Fluids (F) inaktiviert werden, und die Inaktivierung der Viren in dem reaktiven Fluid (F) durch Vermischen des reaktiven Fluids (F) mit einem weiteren Reagenz (R2) oder durch Entfernen des vireninaktivierenden Reagenzes (R1) aus dem reaktiven Fluid (F) gestoppt wird, wodurch ein resultierendes Fluid (RF) erzeugt wird. Vorschlagsgemäß werden die aktiven Viren durch Inkubation des reaktiven Fluids (F) in einer von der ersten Vermischungsanordnung (2) verschiedenen Behälteranordnung (3) mit mehreren gesonderten Behältern (4) inaktiviert, wobei das reaktive Fluid (F) von der ersten Vermischungsanordnung (2) vor der Inkubation zu der Behälteranordnung (3) geleitet wird.

## Beschreibung

Die Erfindung betrifft ein Verfahren zur Inaktivierung von Viren in einem Fluid gemäß dem Oberbegriff von Anspruch 1, ein Verfahren zur Inkubation eines Fluids gemäß dem Oberbegriff von Anspruch 8 sowie eine Inkubationsvorrichtung zur Inkubation eines Fluids gemäß dem Oberbegriff von Anspruch 9.

Auf dem Gebiet der Biotechnologie sind unterschiedliche Verfahren zur Inaktivierung von Viren in einem Fluid bekannt. Eine Inaktivierung von Viren ist etwa bei Prozessen erforderlich, bei denen aktive Viren in bestimmten biopharmazeutischen Produkten, wie etwa Proteinlösungen, enthalten sind. Die Viren können etwa bei dem Prozess im Laufe der Herstellung durch eine externe Quelle, beispielsweise durch für die Fermentation verwendete Ausgangsmaterialien, hinzugefügt oder aber durch interne Quellen, beispielsweise durch die für den Prozess verwendete Zelllinie, eingebracht worden sein. Durch die Inaktivierung der Viren kann eine spätere Infektion von Patienten durch potentielle virale Kontamination in dem Produkt zuverlässig verhindert werden.

Zur Inaktivierung wird zunächst ein mit aktiven Viren belastetes Ausgangsfluid in einer Vermischungsanordnung mit einem vireninaktiverenden Reagenz zu einem reaktiven Fluid vermischt. Die Vermischung umfasst das Hinzufügen des Reagenzes zu dem Ausgangsfluid sowie die zumindest teilweise Homogenisierung. Als Reagenz kommen etwa Mittel in Frage, die eine pH-Wert Absenkung oder Erhöhung des Fluids bewirken, so dass im sauren beziehungsweise basischen Milieu die Inaktivierung der Viren, etwa durch eine Oxidationsreaktion, erfolgt. Es können jedoch auch ebenso alternative vireninaktivierenden Mittel und Chemikalien, die eine entsprechende Inaktivierung bewirken, als Reagenz eingesetzt werden.

Durch Inkubation des reaktiven Fluids werden die aktiven Viren inaktiviert. Die Inkubation des reaktiven Fluids erfolgt dabei für eine erforderliche Zeit, so dass ein bestimmter Anteil der anfangs aktiven Viren inaktiviert wird. Es ist dabei regelmäßig nicht erforderlich, sämtliche aktiven Viren, sondern nur einen gewissen Anteil an aktiven Viren zu inaktiveren.

Nach Inkubation des Fluids wird die Inaktivierung der Viren in dem reaktiven Fluid gestoppt. Dies erfolgt durch Vermischen des reaktiven Fluids mit einem weiteren Reagenz oder durch das Entfernen des vireninaktivierenden Reagenzes aus dem reaktiven Fluid.

So wird etwa bei vorhergehendem Vermischen des Ausgangsfluids mit einem pH-Wert absenkenden oder erhöhenden Reagenz durch das Vermischen des reaktiven Fluids mit einem weiteren Reagenz der pH-Wert wieder erhöht beziehungsweise gesenkt. Bei vorhergehendem Vermischen mit einem alternativen vireninaktivierenden Mittel oder einer alternativen virenaktivierenden Chemikalie als Reagenz wird dieses hingegen wieder aus dem reaktiven Fluid entfernt. Durch das Vermischen mit einem weiteren Reagenz oder durch Entfernen des enthaltenen Reagenzes ergibt sich aus dem reaktiven Fluid ein resultierendes Fluid.

Der bekannte Stand der Technik (US 2013/0260419 A1), von dem die Erfindung ausgeht, betrifft ein derartiges Verfahren. Ein mit aktiven Viren belastetes Fluid wird in einer mehrere Mischer umfassenden Vermischungsanordnung mit einem pH-Wert absenkenden Reagenz vermischt und in der Vermischungsanordnung inkubiert, wodurch die aktiven Viren mit der Zeit inaktiviert werden. Die Inaktivierung wird anschließend durch Vermischen des Fluids mit einem weiteren den pH-Wert erhöhenden Reagenz gestoppt und das resultierende Fluid dadurch erzeugt.

Obschon derartige Verfahren durchaus eine zuverlässige Inaktivierung von Viren in einem Fluid ermöglichen, sind diese wenig flexibel und kaum individuell an unterschiedliche Prozessbedingungen anpassbar. Denn bei derartigen Verfahren sind etwa die Kapazitäten hinsichtlich Fluidmengen und Inkubationszeiten durch die verfügbaren Mischer der Vermischungsanordnung beschränkt, so dass beispielsweise eine Anpassung der Verfahren an sich ändernde Prozessbedingungen, wie etwa größere Fluidmengen oder längere Inkubationszeiten, insoweit nicht ohne weiteres möglich ist.

Der Erfindung liegt daher das Problem zugrunde, die aus dem Stand der Technik bekannten Verfahren derart auszugestalten und weiterzubilden, dass sich ein flexibles und individuell an unterschiedliche Prozessbedingungen anpassbares Verfahren zur Inaktivierung von Viren in einem Fluid ergibt.

Dieses Problem wird bei einem Verfahren gemäß dem Oberbegriff von Anspruch 1 durch die Merkmale des kennzeichnenden Teils von Anspruch 1 gelöst.

Wesentlich ist die grundsätzliche Überlegung, dass die Inkubation des reaktiven Fluids und damit die Inaktivierung der aktiven Viren in mehreren gesonderten Behältern und nicht, wie im Stand der Technik üblich, in der Vermischungsanordnung erfolgt. In jeden der Behälter kann reaktives Fluid gesondert aufgenommen und für eine bestimmte Inkubationszeit unter bestimmten, insbesondere anpassbaren, Inkubationsbedingungen inkubiert werden, während in der Vermischungsanordnung weiteres Ausgangsfluid mit dem vireninaktivierenden Reagenz vermischt werden kann. Hierdurch ist es beispielsweise möglich, dass reaktives Fluid in einem ersten der Behälter inkubiert wird, während in der ersten Vermischungsanordnung bereits zeitgleich weiteres Ausgangsfluid mit dem vireninaktivierenden Reagenz zu weiterem reaktiven Fluid vermischt wird. Das reaktive Fluid aus der ersten Vermischungsanordnung kann anschließend in einen zweiten der Behälter geleitet und inkubiert werden, ohne dass die Inkubation in dem ersten der Behälter hierdurch beeinträchtigt wird. Nach erfolgter Inkubation in dem ersten der Behälter kann dieser entleert und das inkubierte reaktive Fluid weiterverarbeitet werden, während zeitgleich in dem zweiten der Behälter das reaktive Fluid weiterhin inkubiert wird. In der ersten Vermischungsanordnung kann währenddessen bereits eine weitere Vermischung erfolgen.

Im Einzelnen wird ganz allgemein vorgeschlagen, dass die aktiven Viren durch Inkubation des reaktiven Fluids in einer von der ersten Vermischungsanordnung verschiedenen Behälteranordnung mit mehreren gesonderten Behältern inaktiviert werden, wobei das reaktive Fluid von der ersten Vermischungsanordnung vor der Inkubation zu der Behälteranordnung geleitet wird. Durch die räumliche Trennung von Vermischung und Inkubation kann, anders als bei den aus dem Stand der Technik bekannten Verfahren, eine Art Parallelisierung der Verfahrensschritte erfolgen, weshalb sich das Verfahren besonders flexibel und individuell an die unterschiedlichen Prozessbedingungen anpassen lässt. Soll etwa die Inkubationszeit zur Inaktivierung der aktiven Viren verlängert werden, so kann das in den gesonderten Behältern aufgenommene reaktive Fluid ohne weiteres länger inkubiert werden, während zeitgleich die Vermischungsanordnung bereits weiteres Ausgangsfluid und vireninaktivierendes Reagenz zu reaktivem Fluid vermischt und das reaktive Fluid anschließend in einen anderen der Behälter leitet, ohne dass die Inkubation der übrigen Behälter beeinträchtigt wird. Soll etwa eine größere Fluidmenge inkubiert werden, so kann das reaktive Fluid ohne weiteres in mehrere Behälter geleitet werden, insbesondere ohne, dass aufwändige Umkonstruierungen erforderlich werden.

Die Ansprüche 2 und 3 definieren bevorzugte Ausgestaltungen hinsichtlich der Vermischung in der ersten Vermischungsanordnung. Durch die Ausgestaltungen gemäß Anspruch 2 kann in der ersten Vermischungsanordnung eine wechselnde Vermischung von Fluid erfolgen. Insbesondere kann auch diskontinuierlich einströmendes Ausgangsfluid mit dem vireninaktivierenden Reagenz zu dem reaktiven Fluid vermischt werden, indem zeitlich nacheinander folgende Eingangsströme von Ausgangsfluid auf unterschiedliche Mischer und/oder Tangentialflussfilter verteilt werden. So kann beispielsweise in einem ersten Mischer die Vermischung des Ausgangsfluids und des Reagenzes erfolgen während ein zweiter Mischer mit Ausgangsfluid befüllt wird oder etwa das reaktive Fluid des ersten Mischers entleert wird während in dem zweiten Mischer das Ausgangsfluid und das Reagenz vermischt werden. Durch das Erhöhen oder das Absenken des pH-Wertes des Fluids kann eine zuverlässige Vireninaktivierung in dem reaktiven Fluid erfolgen (Anspruch 3). Über das mehrschrittige Erhöhen oder das mehrschrittige Absenken des pH-Wertes gemäß einer vorzugsweisen Ausgestaltung des Anspruchs 3 kann eine stufenweise Einstellung des pH-Wertes erfolgen.

Die Ansprüche 4 und 5 definieren bevorzugte Ausgestaltungen hinsichtlich der Vermischung in einer zweiten Vermischungsanordnung sowie das Entfernen durch eine Trennanordnung. Eine Ausgestaltung gemäß Anspruch 4 mit einer zweiten Vermischungsanordnung ermöglicht eine Vermischung des reaktiven Fluids mit einem weiteren Reagenz nach der Inkubation des Fluids und damit ein Stoppen der Inaktivierung nach erfolgter Inaktivierung der Viren in dem Fluid. Es ist beispielsweise denkbar, dass ein zur Inaktivierung abgesenkter oder erhöhter pH-Wert wieder erhöht oder abgesenkt wird und so eine Neutralisierung des vireninaktivierenden Reagenzes erfolgt. Eine alternative Ausgestaltung gemäß Anspruch 4 mit einer Trennanordnung ermöglicht das Entfernen des vireninaktivierenden Reagenzes aus dem reaktiven Fluid nach der Inkubation des Fluids und damit ein Stoppen der Inaktivierung nach erfolgter Inaktivierung der Viren in dem Fluid. Eine Ausgestaltung gemäß Anspruch 5 mit der zweiten Vermischungsanordnung ermöglicht es insbesondere, auch diskontinuierlich einströmendes reaktives Fluid mit dem weiteren Reagenz zu dem resultierenden Fluid zu vermischen, indem zeitlich nacheinander folgende Eingangsströme auf unterschiedliche Mischer und/oder Tangentialflussfilter verteilt werden. Zusätzlich oder alternativ ermöglicht eine Ausgestaltung gemäß Anspruch 5 einen kontinuierlichen Ausstrom an resultierendem Fluid. So ist es etwa möglich, dass ein erster Mischer entleert und resultierendes Fluid ausströmt, während in einem zweiten Mischer reagierendes Fluid mit einem weiteren Reagenz zu resultierendem Fluid vermischt wird. Sobald das Vermischen in dem zweiten Mischer erfolgt ist, kann der zweite Mischer entleert und das resultierende Fluid ausströmen. Für eine Ausgestaltung gemäß Anspruch 5 mit der Trennanordnung ergeben sich gleichermaßen vorstehend genannte Vorteile.

Die Ausgestaltung gemäß Anspruch 6 betrifft das Befüllen mit reaktivem Fluid aus der ersten Vermischungsanordnung in die Behälter sowie das Entleeren von reaktivem Fluid in die zweite Vermischungsanordnung oder in die Trennanordnung aus den, insbesondere einzeln ansteuerbaren, Behältern. Durch das sequentielle oder zeitgleiche oder zumindest teilweise zeitgleiche Befüllen beziehungsweise Entleeren ist eine besonders einfache Anpassbarkeit des Verfahrens hinsichtlich der Fluidmengen und der Inkubationszeiten möglich.

Die Ausgestaltung gemäß Anspruch 7 ermöglicht eine Übersetzung eines diskontinuierlichen Einströmens von Ausgangsfluid in ein kontinuierliches Ausströmen von resultierendem Fluid nach erfolgter Vireninaktivierung. Hierdurch ist es möglich, das Verfahren auch für Eingangsströme von Ausgangsfluid einzusetzen, die etwa aufgrund eines vorhergehenden diskontinuierlichen Prozesses diskontinuierlich Einströmen, wie einer vorhergehenden Chromatographie, beispielsweise einer *rapid cycling chromatography,* oder einem vorhergehenden Batch-Prozess, und gleichzeitig ein kontinuierliches Ausströmen zu erreichen.

Nach einer weiteren Lehre gemäß Anspruch 8, der eigenständige Bedeutung zukommt, wird ein Verfahren zur Inkubation eines Fluids, vorzugsweise zur Inaktivierung von Viren, mit den Merkmalen gemäß Anspruch 8 vorgeschlagen. Auf alle vorstehenden Ausführungen zum vorschlagsgemäßen Verfahren zur Inaktivierung von Viren darf verwiesen werden, insbesondere hinsichtlich der Ausführungen zum Vermischen eines Ausgangsfluids mit einem, insbesondere ersten, Reagenz zu einem reaktiven Fluid in einer ersten Vermischungsanordnung, zur Inkubation des reaktiven Fluids, zum Vermischen des reaktiven Fluids mit einem weiteren, insbesondere zweiten, Reagenz zu einem resultierenden Fluid, zum Entfernen des, insbesondere ersten, Reagenzes und hinsichtlich der Ausführungen zu den Merkmalen des kennzeichnenden Teils.

Nach einer weiteren Lehre gemäß Anspruch 9, der eigenständige Bedeutung zukommt, wird eine Inkubationsvorrichtung zur Inkubation eines Fluids, insbesondere zur Inaktivierung von Viren, vorgeschlagen. Eine solche Inkubationsvorrichtung kann etwa bei den vorstehend beschriebenen vorschlagsgemäßen Verfahren eingesetzt werden. Es darf insoweit auf alle vorstehenden Ausführungen zu den vorschlagsgemäßen Verfahren verwiesen werden.

Nach einer Ausgestaltung gemäß Anspruch 10 weist die Inkubationsvorrichtung eine zweite Vermischungsanordnung oder eine Trennanordung auf. Über die zweite Vermischungsanordnung ist das reaktive Fluid nach der Inkubation mit einem weiteren die Vireninaktivierung stoppenden Reagenz vermischbar. Über die Trennanordnung ist das vireninaktivierende Reagenz nach der Inkubation aus dem reaktiven Fluid abtrennbar. Hierdurch lässt sich insbesondere der Inaktivierungsprozess stoppen.

Die Ansprüche 11 bis 13 definieren vorteilhafte Ausgestaltungen hinsichtlich der ersten Vermischungsanordnung und der zweiten Vermischungsanordnung und der Trennanordnung. Durch mehrere Mischer oder mehrere Tangentialflussfilter oder einer Kombination aus Mischer und Tangentialflussfilter kann etwa auch diskontinuierlich einströmendes Fluid vermischt werden, indem der oder die Mischer und/oder der oder die Tangentialflussfilter abwechselnd zum Vermischen verwendet werden, oder aber vermischtes Fluid kontinuierlich ausströmen, indem der oder die Mischer abwechselnd entleert und/oder der oder die Tangentialflussfilter abwechselnd durchströmt werden (Anspruch 11). So kann etwa vorgesehen sein, dass in einem ersten Mischer Ausgangsfluid mit einem Reagenz vermischbar ist, während ein zweiter Mischer mit Ausgangsfluid währenddessen befüllbar ist oder aber reaktives Fluid aus dem ersten Mischer entleerbar und Ausgangsfluid mit einem Reagenz in dem zweiten Mischer währenddessen vermischbar ist. Dieselben Vorteile ergeben sich für eine Ausgestaltung der Trennanordnung gemäß Anspruch 11, durch welche etwa auch diskontinuierlich einströmendes Fluid verarbeitet werden kann und durch welche Fluid, aus welchem das Reagenz entfernt wurde, kontinuierlich ausströmen kann. Die modulare Erweiterbarkeit der Inkubationsvorrichtung (Anspruch 12) ermöglicht es, diese besonders flexibel und individuell an den Prozess sowie die Prozessbedingungen anzupassen, insbesondere ohne größere Umkonstruierungen an der Inkubationsvorrichtung vornehmen zu müssen. Durch einen Rezirkulationsabschnitt (Anspruch 13) ist es möglich, das bereits vermischte Fluid in einen Mischer und/oder Tangentialflussfilter der ersten Vermischungsanordnung und/oder in einen Filter und/oder eine Zentrifuge der zweiten Vermischungsanordnung zurückzuführen und zumindest einen Teil des Mischvorgangs, insbesondere wahlweise, oder des Trennvorgangs, also das Entfernen des Reagenzes, insbesondere wahlweise, zu wiederholen.

Nach einer Ausgestaltung gemäß Anspruch 14 weist die Inkubationsvorrichtung einen ersten Verteiler und/oder zweiten Verteiler auf. Über den ersten Verteiler ist das reaktive Fluid aus der ersten Vermischungsanordnung in die Behälter der Behälteranordnung leitbar, über den zweiten Verteiler ist das reaktive Fluid aus den Behältern der Behälteranordnung in die zweite Vermischungsanordnung oder Trennanordnung leitbar. Nach einer weiteren Ausgestaltung gemäß Anspruch 14 lassen sich mit dem ersten Verteiler und/oder mit dem zweiten Verteiler wahlweise Behälter anbinden und/oder davon trennen. Hierdurch lässt sich die Inkubationsvorrichtung ohne große Umkonstruierung flexibel und individuell an den Prozess und an die Prozessbedingungen weiter anpassen, indem etwa Behälter mit den Verteilern verbunden werden, wenn größere Mengen Fluid inkubiert werden sollen und/oder die Inkubationszeit erhöht werden soll, oder etwa Behälter von den Verteilern getrennt werden, wenn kleinere Mengen Fluid inkubiert werden sollen und/oder die Inkubationszeit verringert werden soll. Auch ist es denkbar, Behälter unterschiedlicher Größen zu verbinden.

Nach einer weiteren Ausgestaltung gemäß Anspruch 15 sind die Behälter hängbar ausgebildet, wodurch eine besonders platzsparende Inkubation von Fluid möglich ist. Eine weitere Ausgestaltung gemäß Anspruch 15 sieht vor, dass die Behälter zumindest eine Druckausgleichsöffnung aufweisen. Über die Druckausgleichsöffnung kann ein Druckausgleich in den Behältern erfolgen, so dass beim Befüllen und Entleeren der Behälter mit Fluid kein Über- oder Unterdruck innerhalb der Behälter auftritt.

Nach einer weiteren Ausgestaltung gemäß Anspruch 16 ist oder sind ein Wiegemittel und/oder ein Durchflusssensor und/oder eine Füllstandssonde vorgesehen, über welche die Masse, die Füllmenge und/oder der Füllstand innerhalb der Behälter bestimmbar ist, so dass, insbesondere zu jedem Zeitpunkt, festgestellt werden kann, welche Behälter befüllt und/oder entleert sind.

Nach einer weiteren Ausgestaltung gemäß Anspruch 17 pumpt eine der ersten Vermischungsanordnung in Strömungsrichtung nachgeschaltete Pumpe das reaktive Fluid von der ersten Vermischungsanordnung zu den Behältern und/oder eine den Behältern in Strömungsrichtung nachgeschaltete Pumpe das reaktive Fluid aus den Behältern zu der zweiten Vermischungsanordnung oder der Trennanordnung. Hierdurch kann eine Förderung des jeweiligen Fluids erreicht werden.

Im Folgenden wird die Erfindung anhand einer mehrere Ausführungsbeispiele darstellenden Zeichnung näher erläutert. In der Zeichnung zeigt
- Fig. 1: eine schematische Darstellung einer Vorrichtung zur Durchführung des vorschlagsgemäßen Verfahrens, wobei die Vorrichtung eine erste Vermischungsanordnung mit Mischer und eine zweite Vermischungsanordnung mit Mischer aufweist,
- Fig. 2: eine schematische Darstellung einer weiteren Vorrichtung zur Durchführung des vorschlagsgemäßen Verfahrens, wobei die Vorrichtung eine erste Vermischungsanordnung mit Tangentialflussfilter und eine zweite Vermischungsanordnung mit Tangentialflussfilter aufweist,
- Fig. 3: eine schematische Darstellung einer weiteren Vorrichtung zur Durchführung des vorschlagsgemäßen Verfahrens, wobei die Vorrichtung eine erste Vermischungsanordnung mit Mischer und eine Trennanordnung mit Filter aufweist,
- Fig. 4: eine schematische Darstellung einer weiteren Vorrichtung zur Durchführung des vorschlagsgemäßen Verfahrens, wobei die Vorrichtung eine erste Vermischungsanordnung mit zwei Mischern und eine zweite Vermischungsanordnung mit einem Mischer aufweist,
- Fig. 5: eine schematische Darstellung einer weiteren Vorrichtung zur Durchführung des vorschlagsgemäßen Verfahrens, wobei die Vorrichtung eine erste Vermischungsanordnung mit zwei Mischern und eine zweite Vermischungsanordnung mit zwei Mischern aufweist,
- Fig. 6: eine schematische Darstellung einer weiteren Vorrichtung zur Durchführung des vorschlagsgemäßen Verfahrens, wobei die Vorrichtung eine erste Vermischungsanordnung mit Rezirkulationsabschnitt aufweist,
- Fig. 7: ein Ausführungsbeispiel einer vorschlagsgemäßen Inkubationsvorrichtung zur Durchführung des vorschlagsgemäßen Verfahrens in a) einer ersten Ansicht und b) einer zweiten Ansicht,
- Fig. 8: ein weiteres Ausführungsbeispiel einer vorschlagsgemäßen Inkubationsvorrichtung zur Durchführung des vorschlagsgemäßen Verfahrens in a) einer ersten Ansicht und b) einer zweiten Ansicht,
- Fig. 9: eine Behälteranordnung in a) einer ersten Ausführungsform und b) einer zweiten Ausführungsform.

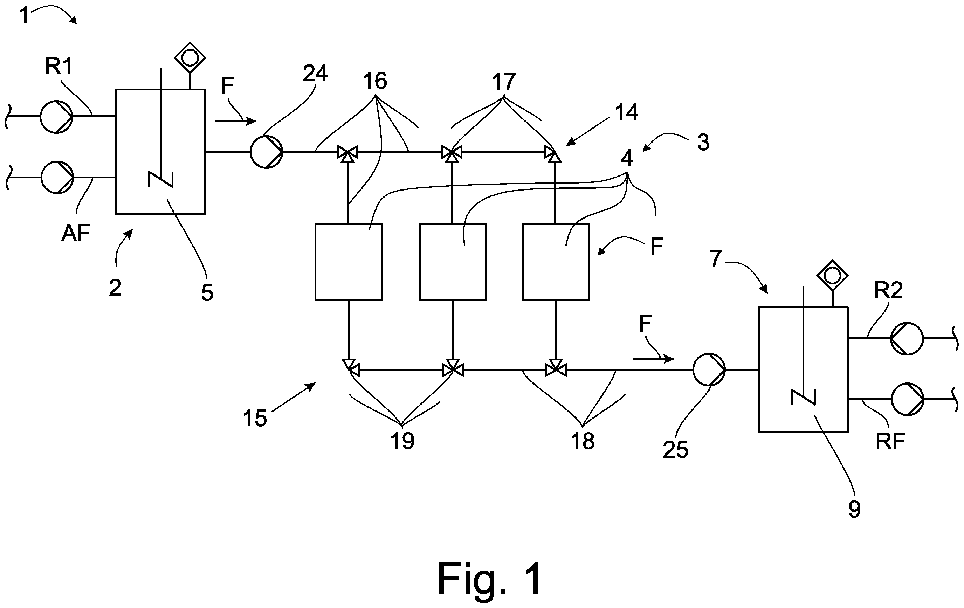

Das vorschlagsgemäß Verfahren zur Inaktivierung von Viren in einem Fluid wird nachfolgend zunächst anhand von Fig. 1, welche ein Ausführungsbeispiel einer Inkubationsvorrichtung 1 darstellt, beschrieben. Das Verfahren ist jedoch keinesfalls auf eine Inkubationsvorrichtung 1 beschränkt, sondern kann auch bei anderen Vorrichtungen und Systemen angewendet werden. Grundsätzlich ist es für jeden der vorschlagsgemäß vorgesehenen und im folgenden näher erläuterten Verfahrensschritte denkbar, dass dieser jeweils automatisiert, insbesondere mittels einer entsprechend konfigurierten Steuerungseinrichtung, initiiert oder durchgeführt wird.

Je nach Art und Herstellungsverfahren des biopharmazeutischen Produkts können unterschiedliche Viren in dem Fluid enthalten sein. Dazu zählen beispielsweise Herpesviren, humane Adenoviren vom Typ 1, Parainfluenzaviren vom Typ 3, das Cache Valley-Virus oder Reoviren vom Typ 3. Prinzipiell ist es außerdem denkbar, dass in einem Fluid Viren unterschiedlicher Art enthalten sind.

Bei dem vorschlagsgemäßen Verfahren zur Inaktivierung von Viren in einem Fluid wird ein mit aktiven Viren belastetes Ausgangsfluid AF in einer ersten Vermischungsanordnung 2 mit einem vireninaktivierenden Reagenz R1 zu einem reaktiven Fluid F vermischt. Das Vermischen in der ersten Vermischungsanordnung 2 umfasst das Hinzufügen des Reagenzes R1 zu dem Ausgangsfluid AF sowie das Mischen zur zumindest teilweisen Homogenisierung der Reagenzes R1 und dem Ausgangsfluid AF.

Die aktiven Viren werden durch Inkubation des reaktiven Fluids F inaktiviert. Die Inkubation erfolgt vorzugsweise nach der Vermischung. Die Inkubation kann unter festlegbaren Inkubationsbedingungen, wie etwa einer bestimmten Temperatur, einem bestimmten Druck, einer bestimmten Zeit, einer bestimmten Bestrahlung des reaktiven Fluids F, etc. erfolgen. Während der Inkubation kann das reaktive Fluid zumindest zeitweise in einem oder mehreren der Behälter 4 verweilen.

"Inaktivierung" im vorliegenden Zusammenhang umfasst, dass zumindest ein gewisser Anteil der anfangs aktiven Viren inaktiviert wird. Es kann insbesondere nur ein Teil der Gesamtheit der aktiven Viren während der Inkubation inaktiviert werden. "Inkubation" umfasst im vorliegenden Zusammenhang insbesondere das Herbeiführen einer Veränderung eines Fluids unter bestimmten Bedingungen, denen das Fluid für eine gewisse Zeit ausgesetzt wird, wie etwa einer Temperatur, einem pH-Wert, etc. Die Bedingungen können dabei über der Zeit variieren.

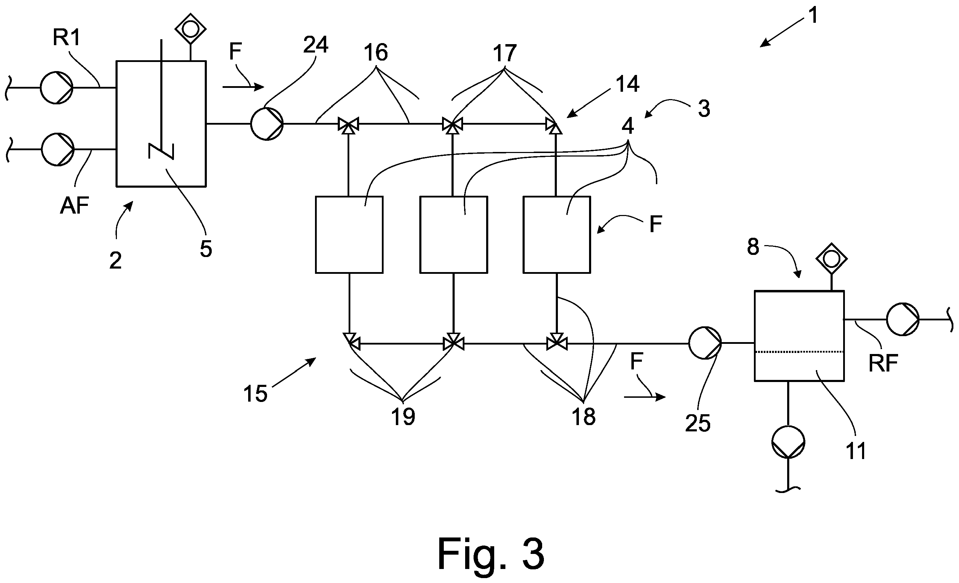

Die Inaktivierung der Viren in dem reaktiven Fluid F wird durch Vermischen des reaktiven Fluids F mit einem weiteren Reagenz R2 gestoppt, siehe Fig. 1. Während das vireninaktivierende Reagenz R1 insoweit die Vireninaktivierung initiieren kann, kann diese durch das weitere Reagenz R2 gestoppt werden. Dies erfolgt vorzugsweise nach der Inkubation. Alternativ hierzu wird die Inaktivierung der Viren in dem reaktiven Fluid F durch Entfernen des vireninaktivierenden Reagenzes R1 aus dem reaktiven Fluid F gestoppt, siehe beispielsweise Fig. 3. Dies erfolgt vorzugsweise nach der Inkubation.

In beiden Fällen wird durch das Stoppen der Inaktivierung ein resultierendes Fluid RF erzeugt. Das resultierende Fluid RF kann etwa in nachfolgenden Prozessen und Verfahren weiterverarbeitet werden.

Wesentlich ist nun, wie es beispielsweise aus Fig. 1 ersichtlich ist, dass die aktiven Viren durch Inkubation des reaktiven Fluids F in einer von der ersten Vermischungsanordnung 2 verschiedenen Behälteranordnung 3 mit mehreren gesonderten Behältern 4 inaktiviert werden, wobei das reaktive Fluid F von der ersten Vermischungsanordnung 2 vor der Inkubation zu der Behälteranordnung 3 geleitet wird. Das Leiten des Fluids F kann dabei definiert erfolgen, etwa durch gängige Mittel zum Leiten von Fluiden, wie etwa durch Leitungen, wie Schläuche oder Rohre. Insbesondere kann das Leiten des Fluids F dadurch erfolgen, dass das Fluid F durch eine oder mehrere Leitungen fließt, vorzugsweise gefördert, insbesondere gepumpt, wird.

Das reaktive Fluid F wird also nicht in der ersten Vermischungsanordnung 2 selbst, sondern in der Behälteranordnung 3 in gesonderten Behältern 4 inkubiert. In der Vermischungsanordnung 2 wird lediglich das vireninaktivierende Reagenz R1 dem Ausgangsfluid AF hinzugefügt und damit vermischt, die Inkubation des reaktiven Fluids F und die damit einhergehende Inaktivierung der Viren erfolgt hingegen in von der Vermischungsanordnung 2 gesonderten, also getrennt ausgebildeten und insbesondere über eine Leitung angeschlossenen, Behältern 4.

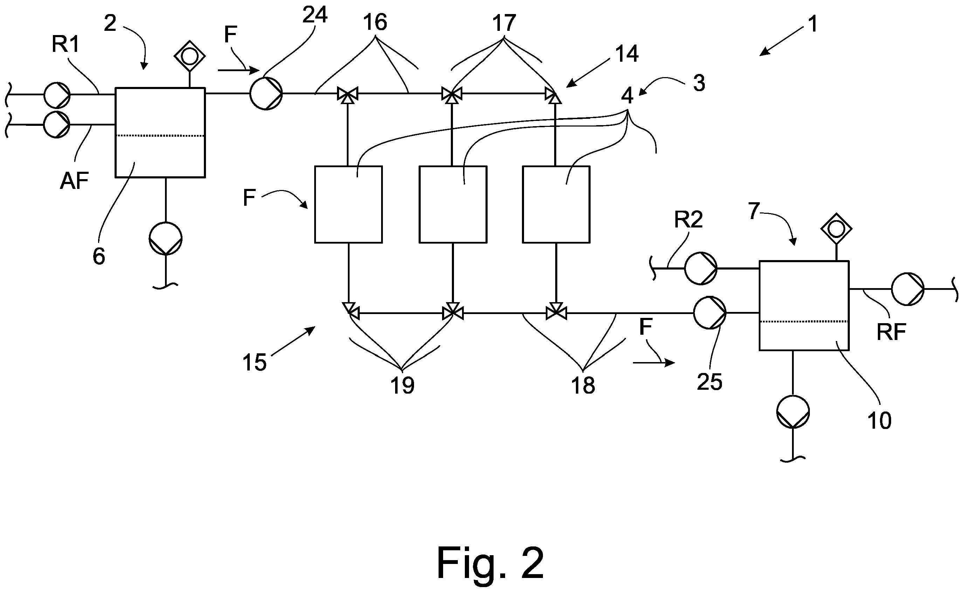

Vorzugsweise weist die erste Vermischungsanordnung zumindest einen Mischer 5 und/oder zumindest einen Tangentialflussfilter 6 auf, welcher oder welche das Ausgangsfluid AF und das vireninaktivierende Reagenz R1 vermischen. Die Vermischung erfolgt sowohl bei einem Mischer 5 als auch bei einem Tangentialflussfilter 6 durch Bewegung des Fluids. Das Ausgangsfluid AF und das vireninaktivierende Reagenz R1 können in den Mischer 5 und/oder den Tangentialflussfilter 6 gesondert, wie etwa in Fig. 1 oder Fig. 2 dargestellt, oder als gemeinsamer Fluidstrom, bei welchem dem Ausgangsfluid AF bereits das vireninaktivierende Reagenz R1 zugefügt, jedoch dieses noch nicht vermischt wurde, geleitet werden.

Allgemein und im vorliegenden Zusammenhang ist ein Mischer 5, 9 vorzugsweise ein dynamischer Mischer, bei welchem ein angetriebener Rührer das in dem Mischer befindliche Fluid derart in Bewegung versetzt, dass eine Vermischung erfolgt, siehe etwa Fig. 1, oder ein statischer Mischer, bei welchem starre oder bewegliche Strömungselemente innerhalb des Mischers 5 das Fluid bei Durchströmen des Mischers derart in Bewegung versetzen, dass eine Vermischung erfolgt, oder eine Kombination aus statischem und dynamischem Mischer.

Im vorliegenden Zusammenhang erfolgt bei einem Tangentialflussfilter 6, 10 vorzugsweise das Vermischen des Fluids, indem etwa interne Strömungen, die beim Durchströmen des Fluids durch den Tangentialflussfilter auftreten und nicht in Richtung der Hauptströmungsrichtung verlaufen, für eine Vermischung sorgen. Ein Tangentialflussfilter wird häufig und vorzugsweise von einem kontinuierlichen Fluidstrom durchströmt. Ein Tangentialflussfilter kann insbesondere eine Doppelfunktion erfüllen, denn dieser kann ein Vermischen des durch den Tangentialflussfilter strömenden Fluids bewirken und gleichzeitig vorzugsweise zumindest einen Teil des durchströmenden Fluids abfiltern. Hierbei wird ein Teil des Fluids durch den Tangentialflussfilter als Permeat abgetrennt und der andere Teil des Fluids durch den Tangentialflussfilter als Retentat abgetrennt.

In dem Ausführungsbeispiel der Fig. 1 weist die erste Vermischungsanordnung 2 beispielsweise einen Mischer 5 auf. Das Ausgangsfluid AF wird mit dem vireninaktivierenden Reagenz R1 in dem Mischer 5 zu dem reaktiven Fluid F vermischt, indem das Ausgangsfluid AF und das vireninaktivierende Reagenz R1 in den Mischer 5 geleitet und vermischt werden. Der Mischer 5 ist, hier und vorzugsweise, von den Behältern 4 verschieden und ist, hier und vorzugsweise, in Strömungsrichtung den Behältern 4 vorausgehend angeordnet.

In dem Ausführungsbeispiel der Fig. 2 weist die Vermischungsanordnung 2 beispielsweise einen Tangentialflussfilter 6 auf. Der Tangentialflussfilter 6 ist vorzugsweise von den Behältern 4 verschieden und ist weiter vorzugsweise in Strömungsrichtung den Behältern 4 vorausgehend angeordnet.

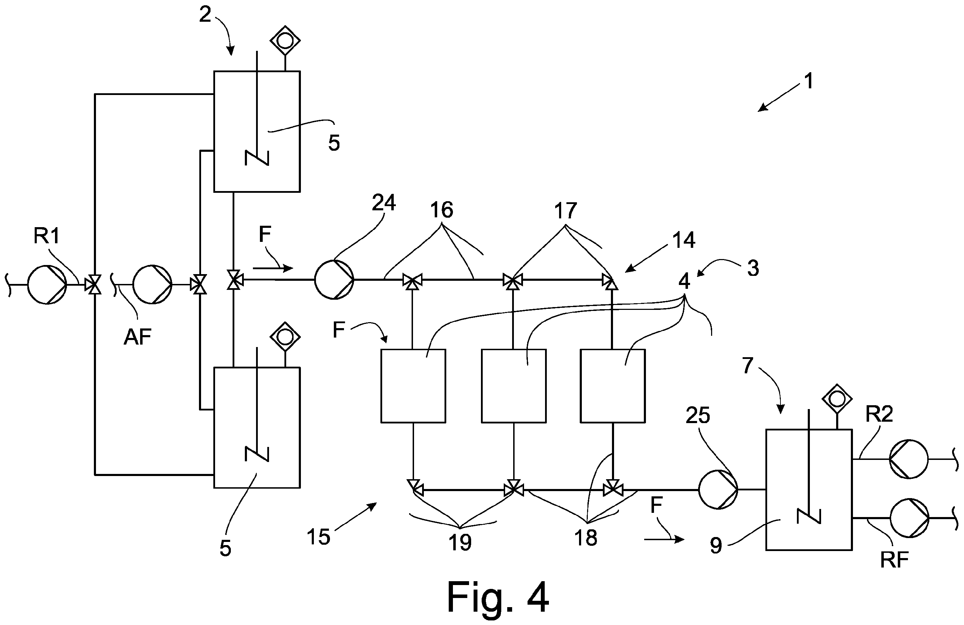

Vorzugsweise und etwa in Fig. 4 dargestellt, wird das Ausgangsfluid AF mit dem vireninaktivierenden Reagenz R1 in zumindest zwei Mischern 5 der ersten Vermischungsanordnung 2 zu dem reaktiven Fluid F vermischt. Die Mischer 5 sind vorzugsweise strömungstechnisch einzeln ansteuerbar. Hierdurch lassen sich die Mischer insbesondere wahlweise und insbesondere in beliebiger Reihenfolge füllen und entleeren. Die Mischer 5 können vorzugsweise sequentiell, also zeitlich nacheinander, oder zeitgleich oder teilweise zeitgleich befüllt werden. Es kann beispielsweise bei dem in Fig. 4 dargestellten Ausführungsbeispiel der Inkubationsvorrichtung 1 vorgesehen sein, dass einer der Mischer 5 mit Ausgangsfluid AF und vireninaktivierendem Reagenz R1 befüllt wird und diese vermischt werden während der andere Mischer 5 erst zeitlich nachfolgend mit Ausgangsfluid AF und vireninaktivierenden Reagenz R1 befüllt wird. Die Entleerung des reaktiven Fluids RF aus den Mischern 5 kann gleichermaßen sequentiell, teilweise zeitgleich oder zeitgleich erfolgen.

Alternativ und vorzugsweise wird das Ausgangsfluid AF mit dem vireninaktivierenden Reagenz R1 in zumindest zwei Tangentialflussfiltern 6 oder zumindest einem Mischer 5 und einem Tangentialflussfilter 6 der ersten Vermischungsanordnung 2 zu dem reaktiven Fluid F vermischt. Diese werden vorzugsweise sequentiell oder zeitgleich oder teilweise zeitgleich befüllt und/oder entleert und/oder durchströmt. Die Tangentialflussfilter 6 oder der Mischer 5 und der Tangentialflussfilter 6 sind vorzugsweise strömungstechnisch einzeln ansteuerbar.

Bei dem vorschlagsgemäßen Verfahren erfolgt vorzugsweise eine Aufkonzentrierung der aktiven Viren in dem reaktiven Fluid F durch den oder die Tangentialflussfilter 6, siehe Fig. 2. Das Ausgangsfluid AF wird mit dem vireninaktivierenden Reagenz R1 durch den Tangentialflussfilter 6 geleitet und vermischt. Das vireninaktivierende Reagenz R1 kann vor dem Tangentialflussfilter 6 dem Ausgangsfluid AF hinzugefügt werden. Alternativ ist es auch denkbar, dass das Reagenz R1 dem Ausgangsfluid AF in dem Tangentialflussfilter 6 hinzugefügt wird. Die Viren verbleiben in dem reaktiven Fluid F vorzugsweise als Retentat, wobei insbesondere das Retentat anschließend inkubiert wird. Bei der Aufkonzentrierung durch den oder die Tangentialflussfilter 6 ergibt sich vorteilhaft, dass kleinere Mengen an reaktivem Fluid F nach der Vermischung zur Inaktivierung der Viren inkubiert werden müssen. Alternativ ist es auch denkbar, dass die Viren in dem reaktiven Fluid F als Permeat verbleiben und insbesondere das Permeat anschließend inkubiert wird.

Bei dem vorschlagsgemäßen Verfahren wird vorzugsweise durch das Vermischen des Ausgangsfluids AF in der ersten Vermischungsanordnung 2 mit dem vireninaktivierenden Reagenz R1, vorzugsweise einer Säure oder einer Base, zu dem reaktiven Fluid F der pH-Wert des Fluids abgesenkt oder erhöht. So kann etwa der pH-Wert durch Hinzugabe einer Säure als Reagenz R1 abgesenkt werden, um ein saures, vireninaktivierendes Milieu zu schaffen. In dem sauren oder basischen Milieu werden die Viren während der Inkubation inaktiviert. Der pH-Wert kann jedoch auch durch Hinzugabe einer Base als Reagenz R2 erhöht werden, um ein basisches, vireninaktivierendes Milieu zu schaffen. Grundsätzlich kann das vireninaktivierende Reagenz R1 eine Säure, eine Base, ein Detergenz, insbesondere Polysorbat, ein Lösemittel und/oder ein Salz oder eine Mischung aus mehreren Säuren, Basen, Detergenzien, Lösemitteln und/oder Salzen sein.

Hier und vorzugsweise erfolgt die Hinzugabe des vireninaktivierenden Reagenzes R1 während des Vermischens in zumindest zwei Schritten. In einem ersten Schritt wird nur ein Teil, vorzugsweise 5% bis 98%, weiter vorzugsweise 50 bis 95%, der erforderlichen Gesamtmenge des vireninaktivierenden Reagenzes R1 mit dem Ausgangsfluid AF vermischt und der pH-Wert des Fluids bestimmt. In einem zweiten Schritt wird der übrige Teil der erforderlichen Gesamtmenge des vireninaktivierenden Reagenzes R1 mit dem Fluid in der ersten Vermischungsanordnung 2 vermischt. Zwischen den Schritten kann vorzugsweise der pH-Wert des Fluids nach zumindest teilweiser Homogenisierung bestimmt werden, etwa durch Messmittel wie Sonden oder Einwegmessmittel wie Einwegsonden.

Insbesondere kann der im ersten Schritt hinzugefügte Teil der erforderlichen Gesamtmenge des Reagenzes R1 abgeschätzt werden. Auf Grundlage des daraufhin gemessenen pH-Wertes kann dann auf die Gesamtmenge des Reagenzes R1 und damit auch auf den restlichen Teil des Reagenzes R1, insbesondere simulativ, rückgeschlossen werden. Vorzugsweise kann das mehrschrittige Vermischen mittels einer künstlichen Intelligenz erfolgen, die aus wiederholten Vermischungsvorgängen lernt, um daraufhin insbesondere die erforderliche Gesamtmenge des Reagenzes R1 abzuschätzen.

Etwa bei dem in Fig. 1 dargestellten Ausführungsbeispiel und vorzugsweise, wird das weitere Reagenz R2 mit dem reaktiven Fluid F in einer zweiten Vermischungsanordnung 7 zu dem resultierenden Fluid RF vermischt. Das Vermischen in der zweiten Vermischungsanordnung 7 umfasst die Hinzugabe des weiteren Reagenzes R2 zu dem reaktiven Fluid F und das Vermischen. Die zweite Vermischungsanordnung 7 ist, hier und vorzugsweise, von der Behälteranordnung 3 verschieden, insbesondere separat, ausgebildet. Die zweite Vermischungsanordnung 7 ist, hier und vorzugsweise, in Strömungsrichtung des Fluids der ersten Vermischungsanordnung 2 und der Behälteranordnung 3 nachfolgend. Die Strömungsrichtung in Fig. 1 ist ausgehend von der Vermischungsanordnung 2 über die Behälteranordnung 3 hin zu der zweiten Vermischungsanordnung 7.

Es ist vorzugsweise denkbar, dass der zweiten Vermischungsanordnung 7 eine Abtrennung in Strömungsrichtung nachfolgend ist. Diese Abtrennung kann etwa durch einen Filter und/oder eine Zentrifuge oder ein anderes Abtrennmittel erfolgen. Das resultierende Fluid RF kann von der zweiten Vermischungsanordnung 7 zu dem Filter und/oder der Zentrifuge und/oder dem anderen Abtrennmittel geleitet werden. Durch die Filter, die Zentrifuge und/oder das andere Abtrennmittel können weitere Bestandteile des resultierenden Fluids RF abgetrennt werden.

Alternativ und vorzugsweise, wird das vireninaktivierende Reagenz R1 aus dem reaktiven Fluid F in einer Trennanordnung 8 entfernt, so wie es exemplarisch in Fig. 3 dargestellt ist. Die Trennanordnung 8 ist vorzugsweise von der Behälteranordnung 3 verschieden und ist vorzugsweise der ersten Vermischungsanordnung 2 und der Behälteranordnung 3 in Strömungsrichtung des Fluids nachfolgen. Die Strömungsrichtung in Fig. 3 ist ausgehend von der Vermischungsanordnung 2 über die Behälteranordnung 3 hin zu der Trennanordnung 8. Die Trennanordnung weist hier und vorzugsweise einen Filter 11 auf, welcher als Tangentialflussfilter ausgebildet ist. Wie vorstehend bereits erörtert, kann ein Tangentialflussfilter ganz allgemein aufgrund dessen Doppelfunktion sowohl zur Vermischung als auch zur Abtrennung eingesetzt werden. Alternativ oder zusätzlich kann die Trennanordnung 8 auch eine Zentrifuge oder andere Abtrennmittel aufweisen, über welche das vireninaktivierende Reagenz R1 abtrennbar ist.

Es sei an dieser Stelle darauf hingewiesen, dass somit der Unterschied zwischen der zweiten Vermischungsanordnung 7 und der Trennanordnung 8 darin besteht, dass, wie oben bereits erläutert, bei der zweiten Vermischungsanordnung 7 die Hinzugabe des weiteren Reagenz R2 zu dem resultierenden Fluid RF und das Mischen erfolgt, wohingegen die Trennanordnung 8, wie oben ebenfalls bereits erläutert, die Entfernung des vireninaktivierenden Reagenz R1 aus dem reaktiven Fluid F zum Ziel hat. Es ist denkbar, dass neben dem Vermischen in der zweiten Vermischungsanordnung 7 zusätzlich auch eine Abtrennung erfolgt und/oder dass neben dem Abtrennen in der Trennanordnung 8 zusätzlich auch eine Vermischung erfolgt.

Bei dem vorschlagsgemäßen Verfahren ist ferner vorzugsweise vorgesehen, dass die Vermischung des reaktiven Fluids F mit dem zweiten Reagenz R2 in der zweiten Vermischungsanordnung 7 mit einem Mischer 9, siehe beispielsweise Fig. 1, oder in einem Tangentialflussfilter 10 erfolgt, siehe beispielsweise Fig. 2.

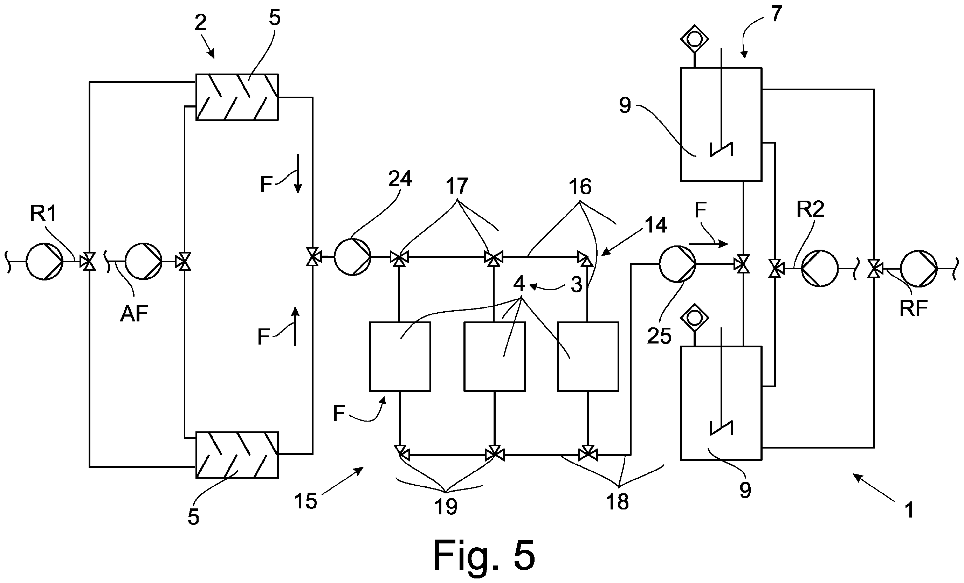

Vorzugsweise weist die zweite Vermischungsanordnung 7 zumindest zwei Mischer 9, wie beispielsweise in Fig. 5 dargestellt, oder zumindest zwei Tangentialflussfilter 10 oder zumindest einen Mischer 9 und einen Tangentialflussfilter 10 auf. Vorzugsweise sind diese von den Behältern 4 verschieden und werden vorzugsweise sequentiell oder zeitgleich oder zumindest teilweise zeitgleich befüllt und/oder entleert und/oder durchströmt. So können etwa der oder die Mischer 9 befüllt und/oder entleert werden und der oder die Tangentialflussfilter 10 durchströmt werden. Der oder die Mischer 9 und/oder der oder die Tangentialflussfilter 10 können einzeln strömungstechnisch angesteuert werden. Dies ist insbesondere wahlweise und insbesondere in beliebiger Reihenfolge denkbar. Jeder Mischer 9, jeder Tangentialflussfilter 10 oder der Mischer 9 und der Tangentialflussfilter 10 kann oder können jeweils durch reaktives Fluid F aus jedem der Behälter 4 befüllt werden. Vorzugsweise strömt durch das sequentielle oder zeitgleiche oder zumindest teilweise zeitgleiche Entleeren oder Durchströmen der Mischer 9 oder der Tangentialflussfilter 10 ein kontinuierlicher Fluidstrom aus der zweiten Vermischungsanordnung 7 aus.

Bei dem vorschlagsgemäßen Verfahren erfolgt vorzugsweise eine Aufkonzentrierung der aktiven Viren in dem resultierenden Fluid RF durch den oder die Tangentialflussfilter 10, siehe Fig. 2. Das reaktive Fluid F wird mit dem weiteren Reagenz R2 durch den Tangentialflussfilter 10 geleitet und vermischt. Das weitere Reagenz R2 kann vor dem Tangentialflussfilter 6 dem reaktiven Fluid F hinzugefügt werden. Alternativ ist es auch denkbar, dass das Reagenz R2 über den Tangentialflussfilter 6 dem reaktiven Fluid F hinzugefügt wird. Die Viren verbleiben in dem resultierenden Fluid RF vorzugsweise als Retentat, wobei insbesondere das Retentat anschließend weiterverarbeitet wird. Alternativ ist es auch denkbar, dass die Viren in dem resultierenden Fluid F als Permeat verbleiben und insbesondere das Permeat anschließend weiterverarbeitet wird.

Alternativ und vorzugsweise, weist die Trennanordnung 8 zumindest zwei Filter 11 oder zwei Zentrifugen oder einen Filter 11 und eine Zentrifuge auf. Diese sind vorzugsweise von den Behältern 4 verschieden. Ferner vorzugsweise werden diese sequentiell oder zeitgleich oder zumindest teilweise zeitgleich befüllt oder durchströmt, wobei vorzugsweise durch das sequentielle oder zeitgleiche oder zumindest teilweise zeitgleiche Durchströmen des oder der Filter 11 und/oder der Zentrifuge oder der Zentrifugen ein kontinuierlicher Fluidstrom aus der Trennanordnung 8 ausströmt. Die Filter 11 können insbesondere durchströmt werden, die Zentrifugen können insbesondere befüllt und entleert werden. Die Filter 11 können vorzugsweise als Tangentialflussfilter ausgebildet sein.

Die Behälter 4 werden weiter vorzugsweise sequentiell, also zeitlich nacheinander, oder zeitgleich oder zumindest teilweise zeitgleich durch reaktives Fluid F aus der ersten Vermischungsanordnung 2 befüllt. Die Behälter 4 können einzeln angesteuert werden. Die Behälter 4 lassen sich vorzugsweise wahlweise und/oder vorzugweise in beliebiger Reihenfolge befüllen. Das reaktive Fluid F aus der ersten Vermischungsanordnung 2 kann in einen der Behälter 4 geleitet oder aber anteilig in mehrere der Behälter 4 geleitet werden.

Hier und vorzugsweise wird das reaktive Fluid F von der ersten Vermischungsanordnung 2 durch einen Verteiler 14 vor der Inkubation zu der Behälteranordnung 3, insbesondere zu den Behältern 4, geleitet. Hierbei kann der Verteiler 14 vorzugsweise als Leitung 16 mit zumindest einem Ventil 17 ausgebildet sein. Die Leitung 16 kann etwa als ein oder mehrere Schläuche und/oder ein oder mehrere Rohre ausgebildet sein. Der Verteiler 14 ermöglicht es vorzugsweise, die Behälter 4 einzeln, insbesondere wahlweise, anzusteuern.

Das reaktive Fluid aus den Behältern 4 wird ferner vorzugsweise sequentiell, also zeitlich nacheinander, oder zeitgleich oder zumindest teilweise zeitgleich in die zweite Vermischungsanordnung 7 oder in die Trennanordnung 8 entleert. Die Behälter 4 können einzeln angesteuert werden. Die Behälter 4 lassen sich vorzugsweise wahlweise und/oder vorzugsweise in beliebiger Reihenfolge entleeren. Das reaktive Fluid F aus einem Behälter 4 kann zu der zweiten Vermischungsanordnung 7 in einen oder mehrere Mischer 9 oder in einen oder mehrere Tangentialflussfilter 10 geleitet werden.

Vorzugsweise wird das reaktive Fluid F in die zweite Vermischungsanordnung 7 oder in die Trennanordnung 8 durch den Verteiler 15 nach der Inkubation von der Behälteranordnung 3, insbesondere aus den Behältern 4, geleitet. Der Verteiler 15 weist hier und vorzugsweise eine Leitung 18 und zumindest ein Ventil 19 auf. Die Leitung 18 kann etwa als ein oder mehrere Schläuche und/oder ein oder mehrere Rohre ausgebildet sein. Vorzugsweise lassen sich über den Verteiler 15 die Behälter 4 einzeln, insbesondere wahlweise ansteuern.

Es ist denkbar, dass das reaktive Fluid F in den Behältern 4 zumindest zeitweise vermischt wird, etwa durch Mischmittel der Behälter 4 und/oder durch Bewegung der Behälter 4. Hierdurch kann etwa die Homogenisierung des reaktiven Fluids F erreicht werden oder die Entmischung des reaktiven Fluids F verhindert werden. Insbesondere wird jedoch in den Behältern 4 kein, insbesondere weiteres, Reagenz hinzugefügt.

Bei dem vorschlagsgemäßen Verfahren ist vorzugsweise vorgesehen, dass das Ausgangsfluid AF in die erste Vermischungsanordnung 2 diskontinuierlich, also zeitlich nicht konstant, einströmt und das resultierende Fluid RF aus der zweiten Vermischungsanordnung 7 oder aus der Trennanordnung 8 kontinuierlich, also zeitlich konstant, ausströmt. "Diskontinuierlich" kann sowohl umfassen, dass Fluid mit einem zeitlich nicht konstanten Volumenstrom ein- oder ausströmt, als auch, dass Fluid nur zu bestimmten Zeiten ein- oder ausströmt. "Kontinuierlich" kann sowohl umfassen, dass Fluid mit einem zeitlich konstanten Volumenstrom ein- oder ausströmt, als auch, dass Fluid dauerhaft, zumindest während des Prozesses, ein- oder ausströmt, insbesondere mit einem zeitlich variierenden oder zeitlich konstanten Volumenstrom.

Es erfolgt dementsprechend vorzugsweise durch das Verfahren eine Art Überführung eines diskontinuierlichen, also zeitlich nicht durchgängig strömenden, insbesondere hinsichtlich des Volumenstroms nicht konstanten, Eingangsstroms von Fluid in einen kontinuierlichen, also zeitlich durchgängig strömenden, insbesondere hinsichtlich des Volumenstroms konstanten, Ausgangsstrom von Fluid.

Vorzugsweise ist es bei dem vorschlagsgemäßen Verfahren denkbar, dass das Ausgangsfluid AF vor dem Einströmen in die erste Vermischungsanordnung 2 ein Chromatografie-Verfahren, insbesondere ein Rapid-Cycling-Chromatografie-Verfahren, durchläuft.

Das vorstehend erörterte Verfahren und dessen Merkmale sowie insbesondere das Inkubationsprinzip lassen sich gleichermaßen auf andere Verfahren, insbesondere Syntheseverfahren, übertragen, bei welchen Fluid inkubiert wird. Daher wird als weitere Lehre, der eigenständige Bedeutung zukommt, ein Verfahren zur Inkubation eines Fluids, vorzugsweise zur Inaktivierung von Viren, vorgeschlagen.

Bei dem vorschlagsgemäßen Verfahren zur Inkubation eines Fluids, vorzugsweise zur Inaktivierung von Viren, wird ein Ausgangsfluid AF in einer ersten Vermischungsanordnung 2 mit einem Reagenz R1 zu einem reaktiven Fluid F vermischt und das reaktive Fluid F inkubiert. Die Inkubation des reaktiven Fluids F wird durch Vermischen des reaktiven Fluids F mit einem weiteren Reagenz R2 oder durch Entfernen des Reagenzes R1 aus dem reaktiven Fluid F gestoppt, wodurch ein resultierendes Fluid RF erzeugt wird. Wesentlich ist, dass das reaktive Fluid F in einer von der ersten Vermischungsanordnung 2 verschiedenen Behälteranordnung 3 mit mehreren gesonderten Behältern 4 inkubiert wird, wobei das reaktive Fluid F von der ersten Vermischungsanordnung 2 vor der Inkubation zu der Behälteranordnung 3 geleitet wird.

Alle vorstehend beschriebenen Merkmale sind im Einzelnen oder in Kombination entsprechend auf das vorschlagsgemäße Verfahren übertragbar. Auf alle Ausführungen zu dem vorschlagsgemäßen Verfahren zur Inaktivierung von Viren in einem Fluid darf insoweit verwiesen werden.

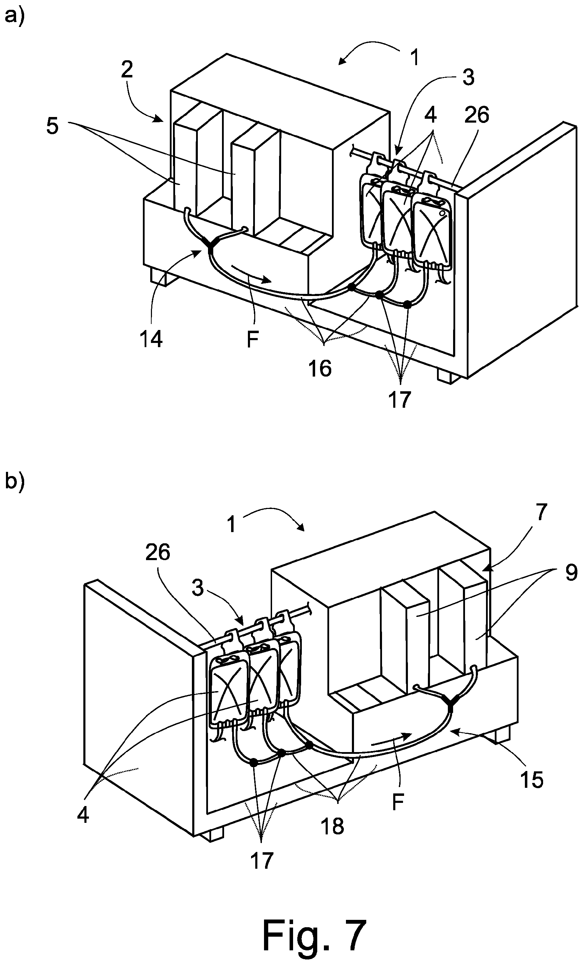

Die vorstehend erörterten vorschlagsgemäßen Verfahren können etwa mit einer vorschlagsgemäßen Inkubationsvorrichtung 1 zur Inkubation eines Fluids, insbesondere zur Inaktivierung von Viren, durchgeführt werden. Neben den Ausführungsbeispielen der Figuren 1 bis 6 zeigen die Figuren 7a), b) sowie 8a), b) eine derartige vorschlagsgemäße Inkubationsvorrichtung 1.

Die Inkubationsvorrichtung 1 zur Inkubation eines Fluids, insbesondere zur Inaktivierung von Viren, weist eine erste Vermischungsanordnung 2 zum Vermischen eines mit aktiven Viren belasteten Ausgangsfluids AF mit einem vireninaktivierenden Reagenz R1 zu einem reaktiven Fluid F auf. Über die erste Vermischungsanordnung 2 ist also ein Ausgangsfluid AF mit einem Reagenz R1 zu einem reaktiven Fluid F vermischbar.

Die Inkubationsvorrichtung 1 weist ferner Behälter 4 zur Inkubation des reaktiven Fluids F auf, siehe beispielsweise Figuren 7a), 7b) und 8a), 8b). In den Behältern 4 ist das reaktive Fluid F inkubierbar, so dass die darin enthaltenen Viren mit der Zeit zumindest zu einem gewissen Anteil inaktiviert werden.

Wesentlich ist, so wie es etwa aus Fig. 1 und den Figuren 7a), b) hervorgeht, dass die Behälter 4 als gesonderte Behälter 4 der von der ersten Vermischungsanordnung 2 verschiedenen Behälteranordnung 3 ausgebildet sind. Die Behälteranordnung 3 ist also von der Vermischungsanordnung 2 getrennt ausgebildet, so dass die Vermischung räumlich getrennt von der Inkubation erfolgen kann.

Die Inkubationsvorrichtung 1 ist, hier und vorzugsweise, als eine Einheit ausgebildet. Alternativ ist es jedoch auch denkbar, dass die Inkubationsvorrichtung 1 als mehrere Einheiten ausgebildet ist.

Die Behälteranordnung 3 ist, hier und vorzugsweise, mit der ersten Vermischungsanordnung 2 strömungstechnisch verbunden. Hierdurch kann reaktives Fluid F von der ersten Vermischungsanordnung 2 zu der Behälteranordnung 3 strömen und die Behälter 4 können befüllt werden.

Die Behälter 4 sind, hier und vorzugsweise, einzeln ausgebildet. Die Behälter 4 sind vorzugsweise einzeln ansteuerbar und sind dadurch insbesondere wahlweise und/oder insbesondere in beliebiger Reihenfolge befüllbar und/oder entleerbar. Insbesondere sind die Behälter 4 sequentiell, also zeitlich nacheinander, oder teilweise zeitgleich oder zeitgleich befüllbar und/oder entleerbar.

Hier und vorzugsweise werden die Behälter 4 während der Inkubation nicht von reaktivem Fluid F durchströmt. Denn während der Inkubation verweilt das reaktive Fluid F zumindest zeitweise in einem der Behälter 4.

Es ist ferner vorzugsweise denkbar, dass die Behälter 4 einbautenfrei ausgebildet sind. Die Behälter 4 enthalten folglich keine Einbauten, insbesondere keine Mischmittel, welche mit dem in den Behältern 4 aufgenommenen reaktive Fluid F in Kontakt stehen. Einbauten im vorliegenden Zusammenhang sind Mittel, die mit dem in den Behältern 4 aufgenommenen reaktiven Fluid F zumindest teilweise in Kontakt stehen und Einfluss auf dieses nehmen, wie etwa Mischmittel, wie Rührer, oder Strömungsbrecher oder Füllkörper oder bestimmte Temperiermittel. Sensoren zur Messung von Eigenschaften des in den Behältern 4 aufgenommenen reaktiven Fluids sind im vorliegenden Zusammenhang nicht als Einbauten zu verstehen. Alternativ ist es ebenso denkbar, dass die Behälter 4 Einbauten aufweisen, wie etwa Mischmittel zur Homogenisierung des in den Behältern 4 aufgenommenen Fluids.

Die Inkubationsvorrichtung 1 weist vorzugsweise eine zweite Vermischungsanordnung 7 zum Vermischen des reaktiven Fluids F mit dem weiteren die Vireninaktivierung stoppenden Reagenz R2 auf, wie es beispielsweise in Fig. 7b) dargestellt ist. Die zweite Vermischungsanordnung 7 ist hier und vorzugsweise von der Behälteranordnung 3 verschieden und weiter vorzugsweise der ersten Vermischungsanordnung 2 und der Behälteranordnung 3 in Strömungsrichtung des Fluids nachfolgend.

Alternativ weist die Inkubationsvorrichtung 1 vorzugsweise eine Trennanordnung 8 zum Entfernen vireninaktivierenden Reagenzes R1 aus dem reaktiven Fluid F auf, siehe etwa Fig. 3. Die Trennanordnung 8 ist vorzugsweise von der Behälteranordnung 3 verschieden und weiter vorzugsweise der ersten Vermischungsanordnung 2 und der Behälteranordnung 3 in Strömungsrichtung des Fluids nachfolgend. Insbesondere in Fällen, in denen die Vireninaktivierung etwa durch das Entfernen des vireninaktivierenden Reagenzes R1 gestoppt wird, hat sich die Inkubationsvorrichtung 1 mit der Trennanordnung 8 bewährt.

Die Trennanordnung 8 umfasst vorzugsweise zumindest ein Abtrennmittel, wie beispielsweise einen Filter 11, wie etwa einen Tangentialflussfilter oder einen Membranfilter, und/oder eine Zentrifuge, wie etwa eine Fließbettzentrifuge (englisch: fluidized bed centrifuge). Über das Abtrennmittel ist das vireninaktivierendes Reagenz R1 aus dem reaktiven Fluid F abtrennbar.

Vorzugsweise und in Figuren 7a), 7b) dargestellt, ist die zweite Vermischungsanordnung 7 konstruktiv gleich zu der ersten Vermischungsanordnung 2 ausgebildet. Es ist alternativ jedoch ebenso denkbar, dass diese konstruktiv unterschiedlich ausgebildet sind.

Die erste Vermischungsanordnung 2 und die zweite Vermischungsanordnung 7 weisen, hier und vorzugsweise, jeweils einen dynamischen Mischer 5, 9 auf. Der dynamische Mischer 5, 9 kann etwa mit einem angetriebenen Rührer, insbesondere Magnetrührer, Stabrührer, etc., ausgestattet sein.

Alternative Ausgestaltungen sind ebenso denkbar, wobei vorzugsweise die erste Vermischungsanordnung 2 und/oder die zweite Vermischungsanordnung 7 zumindest einen statischen Mischer 5, 9, wie etwa in den Figuren 5, 6 und 8a) für die erste Vermischungsanordnung 2 dargestellt, und/oder zumindest einen dynamischen Mischer 5, 9 und/oder zumindest einen Tangentialflussfilter 6, 10, insbesondere einen Single-Pass-Tangentialflussfilter, aufweisen, welcher oder welche vorzugsweise von den Behältern 4 verschieden ist oder sind. In Fig. 2 weist beispielsweise die erste Vermischungsanordnung 2 einen dynamischen Mischer 5 und die zweite Vermischungsanordnung 7 einen Tangentialflussfilter 10 auf.

Bei statischen Mischern 5, 9 kann die Strömungsbewegung des durch den Mischer 5, 9 durchströmenden Fluids eine Mischung bewirken. Statische Mischer 5, 9 werden häufig von einem kontinuierlichen Fluidstrom durchströmt. Bei dynamischen Mischern 5, 9 kann eine Bewegung von Mischelementen eine Bewegung und Mischung des Fluids bewirken. Dynamische Mischer werden häufig diskontinuierlich betrieben, so dass der Fluidstrom diskontinuierlich aus dem dynamischen Mischer 5, 9 strömt.

Bei Tangentialflussfiltern 6, 10 kann die Strömungsbewegung des durch den Tangentialflussfilter 6, 10 durchströmenden Fluids eine Mischung bewirken. Tangentialflussfilter 6, 10 werden häufig von einem kontinuierlichen Fluidstrom durchströmt. Tangentialflussfilter 6, 10 können eine Doppelfunktion erfüllen, denn Tangentialflussfilter 6, 10 können eine Mischung des durch den Tangentialflussfilter 6, 10 strömenden Fluids bewirken und gleichzeitig vorzugsweise zumindest einen Teil des durchströmenden Fluids abfiltern. Hierbei wird ein Teil des Fluids durch den Tangentialflussfilter 6, 10 als Permeat abgetrennt und der andere Teil des Fluids durch den Tangentialflussfilter 6, 10 als Retentat abgetrennt. Durch die Abtrennung in Tangentialflussfiltern 6, 10 kann eine Aufkonzentrierung, insbesondere der aktiven oder inaktivierten Viren, im Retentat erfolgen. Die Aufkonzentrierung kann dazu führen, dass nachfolgend kleinere Volumenströmen des Retentats weiterverarbeitet werden.

Der Tangentialflussfilter 6 und/oder der Tangentialflussfilter 10 kann oder können vorzugsweise als Single-Pass-Tangentialflussfilter ausgebildet sein. Bei Single-Pass-Tangentialflussfiltern strömt das Fluid einmal durch den Filter und wird hierbei gefiltert.

Der Filter 11 der Trennanordnung 8 ist vorzugsweise als Tangentialflussfilter ausgebildet, wobei über den Filter 11 das vireninaktivierende Reagenz R1 abtrennbar ist, siehe Fig. 3. Hierbei ist es denkbar, dass das vireninaktivierende Reagenz R1 als Permeat abgetrennt wird. Alternativ ist es auch denkbar, dass das vireninaktivierende Reagenz R1 als Retentat abgetrennt wird.

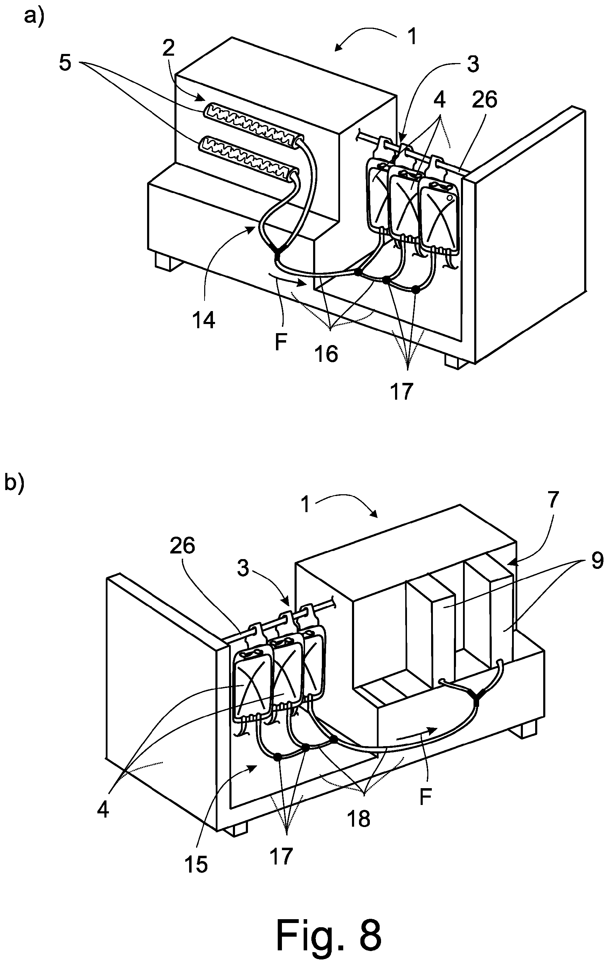

Vorzugsweise und exemplarisch in den Fig. 4, 7a) und 8a) dargestellt, weist die erste Vermischungsanordnung 2 zumindest zwei Mischer 5 auf. Die Mischer 5 sind vorzugsweise von den Behältern 4 verschieden und weiter vorzugsweise strömungstechnisch parallel zueinander angeordnet. Durch die strömungstechnisch parallele Anordnung ist das Ausgangsfluid AF wahlweise sequentiell oder teilweise zeitgleich oder zeitgleich in die Mischer 5 einleitbar. Insbesondere für den Fall von diskontinuierlich einströmendem Ausgangsfluid AF ist so das jeweilige Ausgangsfluid AF in den verschiedenen Mischern 5, insbesondere abwechselnd, vermischbar. Es kann beispielsweise zunächst einer der beiden Mischer 5 mit Ausgangsfluid AF befüllt werden, das vireninaktivierende Reagenz R1 hinzugefügt werden und das Ausgangsfluid AF und das vireninaktivierende Reagenz R1 vermischt werden. Daraufhin kann das Vermischen in dem anderen der beiden Mischer 5 erfolgen. In Fig. 7a) sind die Mischer 5 als dynamische Mischer 5 ausgebildet, in Fig. 8a) sind die Mischer 5 als statische Mischer 5 ausgebildet. Die strömungstechnisch parallele Anordnung ermöglicht vorzugsweise, dass Fluid aus einem Mischer 5 in einen anderen Mischer 5 leitbar ist. Durch die strömungstechnisch parallele Anordnung der Mischer 5 ist zudem eine gewisse Redundanz der Mischer 5 gegeben, so dass auch bei Ausfall eines Mischers 5 die Vermischung erfolgen kann.

Alternativ und vorzugsweise ist es denkbar, die Mischer 5 strömungstechnisch in Reihe zueinander anzuordnen. Hierdurch kann etwa ein Teil der Vermischung in einem der beiden Mischer 5 erfolgen und ein anderer Teil der Vermischung in dem anderen der beiden Mischer 5 erfolgen.

Die erste Vermischungsanordnung 2 kann alternativ auch zwei Tangentialflussfilter 6 oder zumindest einen Mischer 5 und zumindest einen Tangentialflussfilter 6 aufweisen. Diese sind vorzugsweise von den Behältern 4 verschieden und sind vorzugsweise strömungstechnisch parallel oder in Reihe zueinander angeordnet.

Vorzugsweise und exemplarisch in den Fig. 5, Fig. 7b) und Fig. 8b) dargestellt, weist die zweite Vermischungsanordnung 7 zumindest zwei Mischer 9 auf. Die Mischer 9 sind, hier und vorzugsweise, von den Behältern 4 verschieden und, hier und vorzugsweise, strömungstechnisch parallel zueinander angeordnet. Durch die strömungstechnisch parallele Anordnung ist reaktives Fluid F wahlweise sequentiell oder teilweise zeitgleich oder zeitgleich in die Mischer 9 einleitbar. Es ist reaktives Fluid F aus den Behältern 4 in den verschiedenen Mischern 9, insbesondere abwechselnd, vermischbar. Es kann etwa zunächst einer der beiden Mischer 9 mit reaktivem Fluid F befüllt werden, das weitere Reagenz R2 hinzugefügt werden und das reaktive Fluid F und das Reagenz R2 zu dem resultierenden Fluid RF vermischt werden. Daraufhin kann das Vermischen in dem anderen der beiden Mischer 9 erfolgen. Die strömungstechnisch parallele Anordnung ermöglicht vorzugsweise auch, dass Fluid aus einem Mischer 9 in einen anderen Mischer 9 wahlweise leitbar ist. Durch die strömungstechnisch parallele Anordnung der Mischer 9 ist zudem eine gewisse Redundanz der Mischer 9 gegeben, so dass auch bei Ausfall eines Mischers 9 die Vermischung weiterhin erfolgen kann.

Alternativ zu der strömungstechnisch parallelen Anordnung ist es vorzugsweise denkbar, die Mischer 9 strömungstechnisch in Reihe zueinander anzuordnen. Hierdurch kann etwa ein Teil der Vermischung in einem der beiden Mischer 9 und ein anderer Teil der Vermischung in dem anderen der beiden Mischer 9 erfolgen.

Die zweite Vermischungsanordnung 7 kann auch alternativ zwei Tangentialflussfilter 10 oder zumindest einen Mischer 9 und zumindest einen Tangentialflussfilter 10 aufweisen. Diese sind vorzugsweise von den Behältern 4 verschieden und sind weiter vorzugsweise strömungstechnisch parallel oder in Reihe zueinander angeordnet.

Vorzugsweise weist die Trennanordnung 8 zumindest zwei Filter 11 oder zumindest zwei Zentrifugen oder zumindest einen Filter 11 und zumindest eine Zentrifuge aufweist, welche vorzugsweise strömungstechnisch parallel oder in Reihe zueinander angeordnet sind.

Vorzugsweise ist oder sind bei der Inkubationsvorrichtung 1 die erste Vermischungsanordnung 2 und/oder die zweite Vermischungsanordnung 7 derart ausgebildet, dass die erste Vermischungsanordnung 2 und/oder die zweite Vermischungsanordnung 7 wahlweise durch einen oder mehrere Mischer 5, 9, vorzugsweise einen oder mehrere dynamische Mischer oder statische Mischer, und/oder Tangentialflussfilter 6, 10 modular erweiterbar ist oder sind. Bei der Inkubationsvorrichtung 1 der Fig. 7a), b) ist beispielsweise neben den bereits angeschlossenen Mischern 5, 9 noch ein weiterer Mischer 5, 9 anschließbar, so dass die Inkubationsvorrichtung 1 erweitert werden kann, etwa wenn sich die Menge an zu inkubierendem reaktivem Fluid F und/oder die Inkubationszeit verändert. Es ist grundsätzlich auch denkbar, einen oder mehrere der angeschlossenen Mischer 5, 9 und/oder einen oder mehrere der angeschlossenen Tangentialflussfilter 6, 10 wahlweise zu entfernen. Die erste Vermischungsanordnung 2 und/oder die erste Vermischungsanordnung 7 ist oder sind insbesondere werkzeugfrei modular erweiterbar. Hierdurch kann eine Anpassung der Inkubationsvorrichtung 1 ohne großen Aufwand erfolgen.

Vorzugsweise und für den Fall, dass die Inkubationsvorrichtung 1 die Trennanordnung 8 aufweist, ist die Trennanordnung 8 derart ausgebildet, dass diese wahlweise durch einen oder mehrere Filter 11 und/oder durch einen oder mehrere Zentrifugen und/oder durch ein oder mehrere Abtrennmittel modular erweiterbar ist.

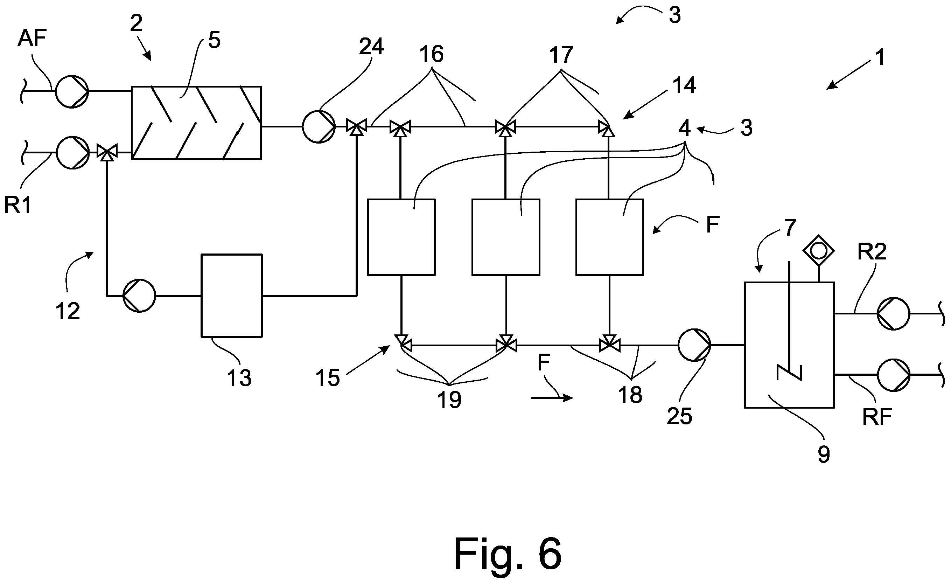

Vorzugsweise weist die erste Vermischungsanordnung 2 und/oder die zweite Vermischungsanordnung 7 einen Rezirkulationsabschnitt 12 auf, über welchen das aus dem zumindest einen Mischer 5, 9 und/oder das aus dem zumindest einen Tangentialflussfilter 6, 10 ausströmende Fluid, insbesondere das Ausgangsfluid AF und/oder das reaktive Fluid F, derart rezirkulierbar ist, dass zumindest ein Teil des Fluids den zumindest einen Mischer 5, 9 und/oder den zumindest einen Tangentialflussfilter 6, 10 mehrfach durchströmt. In dem Ausführungsbeispiel der Fig. 6 weist die erste Vermischungsanordnung 2 den Rezirkulationsabschnitt 12 auf. Der Rezirkulationsabschnitt 12 ermöglicht es, aus dem Mischer 5 strömendes Fluid zumindest teilweise zu rezirkulieren, so dass das Fluid in den Mischer 5 rückführbar ist. Hierdurch kann ein stufenweiser Mischvorgang etwa auch mit nur einem Mischer 5 erfolgen. Die Rezirkulation des Fluids kann insbesondere über die Pumpe 24 erfolgen.

Vorzugsweise weist die Trennanordnung 8 einen Rezirkulationsabschnitt 12 auf, über welchen das aus dem zumindest einen Filter 11 und/oder das aus der zumindest einen Zentrifuge und/oder aus dem zumindest einen Abtrennmittel ausströmende Fluid, insbesondere das reaktive Fluid F, derart rezirkulierbar ist, dass zumindest ein Teil des Fluids den zumindest einen Filter 11 und/oder die zumindest eine Zentrifuge und/oder das zumindest eine Abtrennmittel mehrfach durchströmt.

Es ist denkbar, dass die erste Vermischungsanordnung 2 und/oder die zweite Vermischungsanordnung 7 einen Haltetank 13 aufweist. Der Haltetank 13 ist vorzugsweise in Strömungsrichtung des Fluids dem mindestens einen Mischer 5, 9 oder dem mindestens einen Tangentialflussfilter 6, 10 vorausgehend angeordnet. Alternativ und wie in Fig. 6 gezeigt, kann der Haltetank 13 in Strömungsrichtung des Fluids auch dem mindestens einen Mischer 5, 9 nachgelagert sein. Vorzugsweise und wie ebenfalls in Fig. 6 gezeigt, ist der Haltetank 13 als Teil des Rezirkulationsabschnittes 12 ausgebildet, so dass insbesondere das rezirkulierbare Fluid in den Haltetank 13 rückführbar ist. Über den Haltetank 13 ist vorzugsweise Fluid zwischenspeicherbar, so dass beispielsweise diskontinuierlich einströmendes Ausgangsfluid AF und/oder rezirkuliertes Fluid in dem Haltetank 13 zwischenspeicherbar ist. Der Haltetank 13 ist insbesondere von den Mischern 5, 9 verschieden. Der Haltetank 13 kann vorzugsweise zumindest ein Mischmittel zur Homogenisierung des in dem Haltetank 13 aufgenommenen Fluids aufweisen.

Ferner ist es denkbar, dass die Trennanordnung 8 einen Haltetank 13 aufweist, welcher vorzugsweise als Teil des Rezirkulationsabschnittes 12 ausgebildet ist, so dass insbesondere das rezirkulierbare Fluid in den Haltetank 13 rückführbar ist. Der Haltetank 13 der Trennanordnung 8 kann gleich oder verschieden zu dem vorstehend im Zusammenhang mit der ersten Vermischungsanordnung 2 oder zweiten Vermischungsanordnung 7 beschriebenen Haltetank 13 ausgebildet sein.

Etwa in den Figuren 1, 7a) und 8a) dargestellt und vorzugsweise weist die Inkubationsvorrichtung 1 einen ersten Verteiler 14 zur Verteilung des in der ersten Vermischungsanordnung 2 vermischten reaktiven Fluids F aus der ersten Vermischungsanordnung 2 in die Behälter 4 auf. Über den ersten Verteiler 14 ist Fluid aus der Vermischungsanordnung 2 zu der Behälteranordnung 3, insbesondere den Behältern 4, leitbar. Jeder Behälter 4 ist vorzugsweise über den ersten Verteiler 14 durch reaktives Fluid F eines jeden Mischers 5 und/oder eines jeden Tangentialflussfilters 6 befüllbar. Weist also etwa die erste Vermischungsanordnung 2 mehrere Mischer 5 auf, wie es beispielsweise bei den Inkubationsvorrichtungen 1 der Figuren 7a) und 8a) vorgesehen ist, kann Fluid aus jedem der Mischer 5 wahlweise in jeden der Behälter 4 geleitet werden. Die Behälter 4 können damit strömungstechnisch einzeln ansteuerbar sein.

Es ist alternativ oder zusätzlich denkbar, dass die Inkubationsvorrichtung 1 einen zweiten Verteiler 15 zur Verteilung des inkubierten reaktiven Fluids F aus den Behältern 4 aufweist. Über den zweiten Verteiler 15 ist Fluid aus der Behälteranordnung 3, insbesondere den Behältern 4, zu der zweiten Vermischungsanordnung 7 leitbar. Vorzugsweise ist jeder Mischer 9 und/oder Tangentialflussfilter 10 durch reaktives Fluid F eines jeden Behälters 4 befüllbar oder durchströmbar. Weist also etwa die zweite Vermischungsanordnung 7 mehrere Mischer 9 auf, wie es beispielsweise bei den Inkubationsvorrichtungen 1 in den Figuren 7b) und 8b) vorgesehen ist, kann Fluid aus jedem der Behälter 4 wahlweise in jeden der Mischer 9 geleitet werden. Die Mischer 9 und/oder die Tangentialflussfilter 10 sind vorzugsweise entsprechend strömungstechnisch einzeln ansteuerbar.

Es ist alternativ oder zusätzlich denkbar, dass über den zweiten Verteiler 15 Fluid aus der Behälteranordnung 3, insbesondere den Behältern 4, zu der Trennanordnung 8 leitbar ist. Vorzugsweise ist jeder Filter 11 und/oder jede Zentrifuge und/oder jedes Abtrennmittel durch reaktives Fluid F eines jeden Behälters 4 befüllbar oder durchströmbar. Weist also etwa die Trennanordnung 8 mehrere Mischer 9 auf, kann Fluid aus jedem der Behälter 4 wahlweise in jeden der Filter 11 geleitet werden. Die Filter 11 und/oder die Zentrifugen sind vorzugsweise entsprechend strömungstechnisch einzeln ansteuerbar.

Der Verteiler 14 und der Verteiler 15 sind hier und vorzugsweise über die Behälter 4 strömungstechnisch miteinander verbunden. Durch die räumliche Trennung kann eine Kontamination der inkubierten reaktiven Fluids F in dem Verteiler 15 mit dem bislang nicht inkubierten reaktiven Fluid F in dem Verteiler 14 vermieden werden.

Vorzugsweise und wie in den Figuren 1, 7a) und b) sowie 8a) und b) dargestellt, ist der erste Verteiler 14 und/oder zweite Verteiler 15 als Leitung 16, 18 mit mehreren Ventilen 17, 19 oder als Leitung 16, 18 mit einem Mehrwegeventil, mit insbesondere mehr als drei möglichen Schaltstellungen, ausgebildet. Die Ventile 17, 19 sind hier und vorzugsweise als 3/2-Wege-Ventile ausgebildet. Ein 3/2-Wege-Ventil weist drei Anschlüsse auf und zwei Schaltstellungen. Die Leitung 16, 18 ist hier und vorzugsweise als mehrere Abschnitte aufweisende Leitung 16, 18 ausgebildet sein. Die Leitung 16, 18 kann vorzugsweise als Rohr- oder Schlauchleitung ausgebildet sein.

Vorzugsweise ist der erste Verteiler 14 und/oder der zweite Verteiler 15 derart ausgebildet, dass wahlweise Behälter 4 mit dem ersten und/oder zweiten Verteiler 14, 15, insbesondere werkzeuglos, verbindbar und/oder trennbar sind. Bei den Inkubationsvorrichtungen 1 der Figuren 7a), b) und 8a), b) weisen beispielsweise der erste Verteiler 14 und der zweite Verteiler 15 jeweils eine freie Verbindungsstelle aus, so dass ein weiterer Behälter 4 mit den Verteilern 14, 15 verbindbar ist. Hierdurch lässt sich etwa die Menge an inkubierbarem Fluid und/oder die Inkubationszeit ohne weitere konstruktive Änderungen anpassen. Hier und vorzugsweise sind die Behälter 4 modular ausgebildet, so dass diese wahlweise mit den Verteilern 14, 15, insbesondere werkzeuglos, verbindbar und/oder trennbar sind.

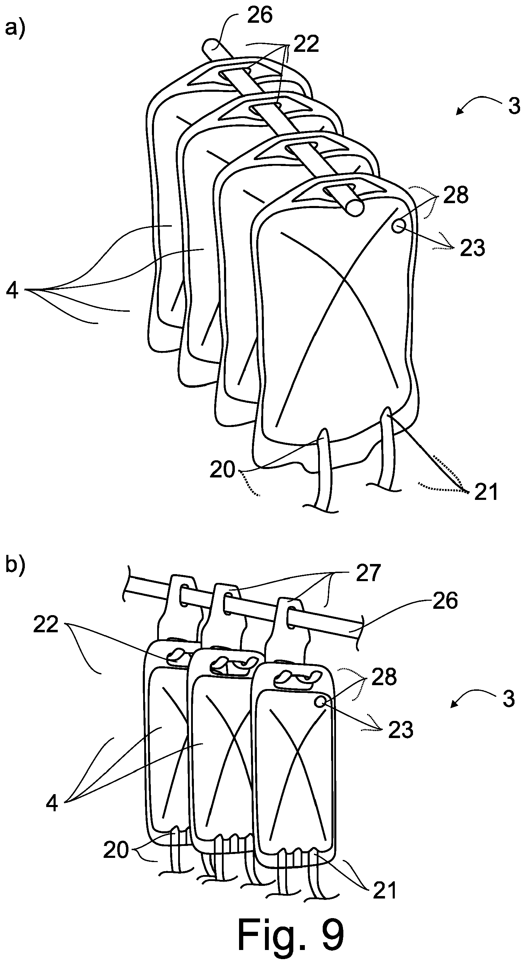

Es hat sich bewährt, wie etwa in den Figuren 9a) und 9b) dargestellt, dass die Behälter 4 jeweils einen Behältereinlass 20 und einen Behälterauslass 21 aufweisen. Die Behälter 4 können über die Behältereinlässe 20, insbesondere über den ersten Verteiler 14, mit reaktivem Fluid F aus der ersten Vermischungsanordnung 2 befüllbar und über die Behälterauslässe 21, insbesondere über den zweiten Verteiler 15, in die zweite Vermischungsanordnung 7 oder die Trennanordnung 8 entleerbar sein.

Es ist vorzugsweise denkbar, dass die Behälter 3 und/oder der oder die Mischer 5 und/oder der oder die Mischer 9 und/oder der oder die Tangentialflussfilter 6 und/oder der oder die Tangentialflussfilter 10 als Einwegprodukte ausgebildet sind. Durch den regelmäßigen Tausch der Bauteile können Kontaminationen vermieden werden.

Vorzugsweise sind die Behälter 4 hängbar ausgebildet, siehe Figuren 7a), b), 8a), b) sowie 9a), b). Hierdurch kann eine platzsparende Inkubation in den Behältern 4 erfolgen, da diese entsprechend platzsparend gelagert werden. Es hat sich insbesondere bewährt, dass die Behälter 4 jeweils nach Art einer hängbaren Tasche oder, wie es beispielhaft in den Figuren 9a) und b) dargestellt ist, jeweils nach Art eines hängbaren Beutels, insbesondere nach Art eines Infusionsbeutels, ausgebildet sind. Die Behälter 4 weisen weiter vorzugsweise und in den Figuren 9a), b) dargestellt jeweils eine Öse 22 auf, über welche der jeweilige Behälter 4 hängbar ist. Die Inkubationsvorrichtung 1 kann etwa eine Hängestange 26 aufweisen, an welche die Behälter 4 über die Öse 22 direkt aufgereiht angeordnet, insbesondere angehangen, werden, wie es beispielsweise in Fig. 9a) dargestellt ist. Es ist auch denkbar, dass an die Hängestange 26 mehrere Haken 27 angeordnet, insbesondere angehangen, sind und an die Haken 27 die Behälter 4 angeordnet, insbesondere angehangen, werden, wie es beispielsweise in Fig. 9b) dargestellt ist. Die Behälter 4 können vorzugsweise aus flexiblem Kunststoff gefertigt sein.

Vorzugsweise und in den Figuren 9a), b) dargestellt, weisen die Behälter 4 jeweils zumindest eine Druckausgleichsöffnung 23 zum Druckausgleich in dem jeweiligen Behälter 4 auf. Beim Einströmen und Ausströmen von reaktiven Fluid F in die Behälter 4 kann die resultierende Druckänderung in dem jeweiligen Behälter 4 über die jeweilige Druckausgleichsöffnung 23 ausgeglichen werden, indem beispielsweise Umgebungsluft oder Schutzgas über die Druckausgleichsöffnung 23 in den Behälter 4 einströmt oder Gas aus dem Behälter 4 über die Druckausgleichsöffnung 23 ausströmt. Um Kontaminationen des in den Behältern 4 aufgenommenen Fluids zu verhindern, etwa mit Verunreinigungen in der Umgebungsluft, weist die Druckausgleichsöffnung 23 vorzugsweise einen Öffnungsfilter 28 auf. Alternativ zu einer Druckausgleichsöffnung 23, die zum Einströmen und Ausströmen ausgebildet ist, ist es ebenso denkbar mehrere separate Druckausgleichsöffnungen 23 je Behälter 4 vorzusehen, so dass etwa durch eine erste Druckausgleichsöffnung 23 Umgebungsluft oder Schutzgas einströmen und über eine zweite Druckausgleichsöffnung 23 Gas aus dem Behälter 24 ausströmen kann. Die Druckausgleichsöffnungen 23 können insbesondere ein Ventil aufweisen.

Es hat sich darüber bewährt, dass die Inkubationsvorrichtung 1 ein Wiegemittel, vorzugsweise eine Wägezelle und/oder einen Kraftaufnehmer, insbesondere zum Wiegen des reaktiven Fluids F in den Behältern 4, und/oder einen Durchflusssensor zur Regulierung der Füllmenge an reaktivem Fluid F in den Behältern 4 und/oder eine Füllstandsonde zum Erfassen des Füllstands an reaktivem Fluid F in den Behältern 4 aufweist. Über das Wiegemittel kann die Masse des reaktiven Fluids in dem Behälter 4 bestimmt werden, indem diese etwa gewogen wird. Über den Durchflusssensor kann der jeweilige Fluidstrom in einem der Behälter 4 gemessen werden. Über die Füllstandsonde kann der jeweilige Füllstand des reaktiven Fluids F in einem der Behälter 4 gemessen werden. Durch die vorgeschlagene Ausgestaltung mit Wiegemittel und/oder Durchflusssensor und/oder Füllstandsonde kann die Menge an reaktivem Fluid F in dem jeweiligen Behälter 4 bestimmt werden. Vorzugsweise kann der Durchflusssensor auch dazu eingerichtet sein, den Fluiddruck des durch den Durchflusssensor durchströmenden Fluids zu bestimmen. Vorzugsweise weist die Inkubationsvorrichtung 1 einen ersten Durchflussmesser und einen zweiten Durchflussmesser auf, wobei über den ersten Durchflussmesser der in die Behälter 4 einströmende Fluidstrom und den zweiten Durchflussmesser der aus den Behältern 4 ausströmende Fluidstrom erfassbar ist.

Etwa in Fig. 1 bis 6 dargestellt und vorzugsweise weist die Inkubationsvorrichtung 1 eine der ersten Vermischungsanordnung 2 in Strömungsrichtung nachgeschaltete Pumpe 24, insbesondere eine Peristaltikpumpe, zum Pumpen des in der ersten Vermischungsanordnung 2 vermischten reaktiven Fluids F zu den Behältern 4 und/oder eine den Behältern 4 in Strömungsrichtung nachgeschaltete Pumpe 25, insbesondere eine Peristaltikpumpe, zum Pumpen des inkubierten reaktiven Fluids F zu der zweiten Vermischungsanordnung 7 oder zu der Trennanordnung 8 auf. Das reaktive Fluid F ist damit zu den Behältern 5 über die Pumpe 24 förderbar und/oder aus den Behältern 5 über die Pumpe 25 förderbar. Hierdurch lässt sich reaktives Fluid F zu oder aus beliebig vielen Behältern 4 über jeweils eine der Pumpen 24, 25 fördern. Vorzugsweise lässt sich über jeweils eine der Pumpen 24, 25 das Fluid durch den ersten Verteiler 14 und/oder durch den zweiten Verteiler 15 fördern. Es können alternativ auch mehrere Pumpen 24, 25 vorgesehen sein. Die Pumpe 24, 25 oder die Pumpen 24, 25 können vorzugsweise als Peristaltikpumpe oder Peristaltikpumpen ausgebildet sein.

Die vorschlagsgemäße Inkubationsvorrichtung 1 ermöglicht die Inkubation eines Fluids, insbesondere zur Inaktivierung von Viren. Grundsätzlich ist die Inkubationsvorrichtung 1 jedoch auch für andere Verfahren, bei denen eine Inkubation eines Fluids erfolgt, geeignet, wie insbesondere für unterschiedliche Syntheseverfahren.

## Patentansprüche

1. Verfahren zur Inaktivierung von Viren in einem Fluid, bei welchem ein mit aktiven Viren belastetes Ausgangsfluid (AF) in einer ersten Vermischungsanordnung (2) mit einem vireninaktivierenden Reagenz (R1) zu einem reaktiven Fluid (F) vermischt wird und die aktiven Viren durch Inkubation des reaktiven Fluids (F) inaktiviert werden, und die Inaktivierung der Viren in dem reaktiven Fluid (F) durch Vermischen des reaktiven Fluids (F) mit einem weiteren Reagenz (R2) oder durch Entfernen des vireninaktivierenden Reagenzes (R1) aus dem reaktiven Fluid (F) gestoppt wird, wodurch ein resultierendes Fluid (RF) erzeugt wird,
**dadurch gekennzeichnet,**
**dass** die aktiven Viren durch Inkubation des reaktiven Fluids (F) in einer von der ersten Vermischungsanordnung (2) verschiedenen Behälteranordnung (3) mit mehreren gesonderten Behältern (4) inaktiviert werden, wobei das reaktive Fluid (F) von der ersten Vermischungsanordnung (2) vor der Inkubation zu der Behälteranordnung (3) geleitet wird.

2. Verfahren nach Anspruch 1, **dadurch gekennzeichnet, dass** das Ausgangsfluid (AF) mit dem vireninaktivierenden Reagenz (R1) in zumindest zwei Mischern (5) oder in zumindest zwei Tangentialflussfiltern (6) oder zumindest einem Mischer (5) und einem Tangentialflussfilter (6) der ersten Vermischungsanordnung (2) zu dem reaktiven Fluid (F) vermischt wird, welche vorzugsweise sequentiell oder zeitgleich oder teilweise zeitgleich befüllt und/oder entleert und/oder durchströmt werden.

3. Verfahren nach Anspruch 1 oder 2, **dadurch gekennzeichnet, dass** durch das Vermischen des Ausgangsfluids (AF) in der ersten Vermischungsanordnung (2) mit dem vireninaktivierenden Reagenz (R1), vorzugsweise einer Säure oder einer Base, zu dem reaktiven Fluid (F) der pH-Wert des Fluids abgesenkt oder erhöht wird, vorzugsweise, dass die Hinzugabe des vireninaktivierenden Reagenzes (R1) während des Vermischens in zumindest zwei Schritten erfolgt, wobei in einem ersten Schritt nur ein Teil, vorzugsweise 5% bis 98%, weiter vorzugsweise 50 bis 95%, der erforderlichen Gesamtmenge des vireninaktivierenden Reagenzes (R1) mit dem Ausgangsfluid (AF) vermischt und der pH-Wert des Fluids bestimmt wird und in einem zweiten Schritt der übrige Teil der erforderlichen Gesamtmenge des vireninaktivierenden Reagenzes (R1) mit dem Fluid in der ersten Vermischungsanordnung (2) vermischt wird.

4. Verfahren nach einem der vorhergehenden Ansprüche, **dadurch gekennzeichnet, dass** das weitere Reagenz (R2) mit dem reaktiven Fluid (F) in einer zweiten Vermischungsanordnung (7) zu dem resultierenden Fluid (RF) vermischt wird, welche vorzugsweise von der Behälteranordnung (3) verschieden ist und vorzugsweise der ersten Vermischungsanordnung (2) und der Behälteranordnung (3) in Strömungsrichtung des Fluids nachfolgend ist, oder, dass das vireninaktivierende Reagenz (R1) aus dem reaktiven Fluid (F) in einer Trennanordnung (8) entfernt wird, welche vorzugsweise von der Behälteranordnung (3) verschieden ist und vorzugsweise der ersten Vermischungsanordnung (2) und der Behälteranordnung (3) in Strömungsrichtung des Fluids nachfolgend ist.

5. Verfahren nach Anspruch 4, **dadurch gekennzeichnet, dass** die zweite Vermischungsanordnung (7) zumindest zwei Mischer (9) oder zumindest zwei Tangentialflussfilter (10) oder zumindest einen Mischer (9) und einen Tangentialflussfilter (10) aufweist, welche vorzugsweise von den Behältern (4) verschieden sind und vorzugsweise sequentiell oder zeitgleich oder zumindest teilweise zeitgleich befüllt und/oder entleert und/oder durchströmt werden, vorzugsweise, dass durch das sequentielle oder zeitgleiche oder zumindest teilweise zeitgleiche Entleeren oder Durchströmen der Mischer (9) oder der Tangentialflussfilter (10) ein kontinuierlicher Fluidstrom aus der zweiten Vermischungsanordnung (7) ausströmt, oder,
dass die Trennanordnung (8) zumindest zwei Filter (11) oder zwei Zentrifugen oder einen Filter (11) und eine Zentrifuge aufweist, welche vorzugsweise von den Behältern (4) verschieden sind und welche vorzugsweise sequentiell oder zeitgleich oder zumindest teilweise zeitgleich durchströmt werden, vorzugsweise, dass durch das sequentielle oder zeitgleiche oder zumindest teilweise zeitgleiche Durchströmen der Filter (11) oder der Zentrifugen oder des Filters und der Zentrifuge ein kontinuierlicher Fluidstrom aus der Trennanordnung (8) ausströmt.

6. Verfahren nach einem der vorhergehenden Ansprüche, **dadurch gekennzeichnet, dass** die Behälter (4) sequentiell oder zeitgleich oder zumindest teilweise zeitgleich durch reaktives Fluid (F) aus der ersten Vermischungsanordnung (2) befüllt werden und/oder das reaktive Fluid (F) aus den Behältern (4) sequentiell oder zeitgleich oder zumindest teilweise zeitgleich in die zweite Vermischungsanordnung (7) oder in die Trennanordnung (8) entleert wird.

7. Verfahren nach einem der vorhergehenden Ansprüche, **dadurch gekennzeichnet, dass** das Ausgangsfluid (AF) in die erste Vermischungsanordnung (2) diskontinuierlich oder semi-kontinuierlich einströmt und das resultierende Fluid (RF) aus der zweiten Vermischungsanordnung (7) kontinuierlich ausströmt.

8. Verfahren zur Inkubation eines Fluids, vorzugsweise zur Inaktivierung von Viren, bei welchem ein Ausgangsfluid (AF) in einer ersten Vermischungsanordnung (2) mit einem Reagenz (R1) zu einem reaktiven Fluid (F) vermischt wird und das reaktive Fluid (F) inkubiert wird und die Inkubation des reaktiven Fluids (F) durch Vermischen des reaktiven Fluids (F) mit einem weiteren Reagenz (R2) oder durch Entfernen des Reagenzes (R1) aus dem reaktiven Fluid (F) gestoppt wird, wodurch ein resultierendes Fluid (RF) erzeugt wird,
**dadurch gekennzeichnet,**
**dass** das reaktive Fluid (F) in einer von der ersten Vermischungsanordnung (2) verschiedenen Behälteranordnung (3) mit mehreren gesonderten Behältern (4) inkubiert wird, wobei das reaktive Fluid (F) von der ersten Vermischungsanordnung (2) vor der Inkubation zu der Behälteranordnung (3) geleitet wird.

9. Inkubationsvorrichtung zur Inkubation eines Fluids, insbesondere zur Inaktivierung von Viren in dem Fluid, mit einer ersten Vermischungsanordnung (2) zum Vermischen eines mit aktiven Viren belasteten Ausgangsfluid (AF) mit einem vireninaktivierenden Reagenz (R1) zu einem reaktiven Fluid (F), und Behältern (4) zur Inkubation des reaktiven Fluids (F),
**dadurch gekennzeichnet,**
**dass** die Behälter (4) als gesonderte Behälter (4) einer von der ersten Vermischungsanordnung (2) verschiedenen Behälteranordnung (3) ausgebildet sind.

10. Inkubationsvorrichtung nach Anspruch 9, **dadurch gekennzeichnet, dass** die Inkubationsvorrichtung (1) eine zweite Vermischungsanordnung (7) zum Vermischen des reaktiven Fluids (F) mit einem weiteren die Vireninaktivierung stoppenden Reagenz (R2) aufweist, welche vorzugsweise von der Behälteranordnung (3) verschieden ist, oder, dass die Inkubationsvorrichtung (1) eine Trennanordnung (8) zum Entfernen des vireninaktivierenden Reagenzes (R1) aus dem reaktiven Fluid (F) aufweist, welche vorzugsweise von der Behälteranordnung (3) verschieden ist, vorzugsweise, dass die zweite Vermischungsanordnung (7) und/oder die Trennanordnung (8) der ersten Vermischungsanordnung (2) und der Behälteranordnung (3) in Strömungsrichtung des Fluids nachfolgend ist.

11. Inkubationsvorrichtung nach Anspruch 9 oder 10, **dadurch gekennzeichnet, dass** die erste Vermischungsanordnung (2) und/oder die zweite Vermischungsanordnung (7) zumindest zwei Mischer (5, 9) oder zumindest zwei Tangentialflussfilter (6, 10) oder zumindest einen Mischer (5, 9) und einen Tangentialflussfilter (6, 10) aufweist, welche vorzugsweise von den Behältern (4) verschieden sind und vorzugsweise strömungstechnisch parallel oder in Reihe zueinander angeordnet sind, und/oder,
dass die Trennanordnung (8) zumindest zwei Filter (11) oder zumindest zwei Zentrifugen oder zumindest einen Filter (11) und zumindest eine Zentrifuge aufweist, welche vorzugsweise strömungstechnisch parallel oder in Reihe zueinander angeordnet sind.

12. Inkubationsvorrichtung nach einem der Ansprüche 9 bis 11, **dadurch gekennzeichnet, dass** die erste Vermischungsanordnung (2) und/oder die zweite Vermischungsanordnung (7) derart ausgebildet ist, dass diese wahlweise durch einen oder mehrere statische Mischer (5, 9) und/oder dynamische Mischer (5, 9) und/oder Tangentialflussfilter (6, 10) modular erweiterbar ist, und/oder,
dass die Trennanordnung (8) derart ausgebildet ist, dass diese wahlweise durch einen oder mehrere Filter (11) und/oder durch einen oder mehrere Zentrifugen modular erweiterbar ist.

13. Inkubationsvorrichtung nach einem der Ansprüche 9 bis 12, **dadurch gekennzeichnet, dass** die erste Vermischungsanordnung (2) und/oder die zweite Vermischungsanordnung (7) einen Rezirkulationsabschnitt (12) aufweist, über welchen das aus dem zumindest einen Mischer (5, 9) und/oder das aus dem zumindest einen Tangentialflussfilter (6, 10) ausströmende Fluid derart rezirkulierbar ist, dass zumindest ein Teil des Fluids den zumindest einen Mischer (5, 9) und/oder den zumindest einen Tangentialflussfilter (6, 10) mehrfach durchströmt, und/oder,
dass die Trennanordnung (8) einen Rezirkulationsabschnitt (12) aufweist, über welchen das aus dem zumindest einen Filter (11) und/oder das aus der zumindest einen Zentrifuge ausströmende Fluid derart rezirkulierbar ist, dass zumindest ein Teil des Fluids den zumindest einen Filter (11) und/oder die zumindest eine Zentrifuge mehrfach durchströmt.

14. Inkubationsvorrichtung nach einem der Ansprüche 9 bis 13, **dadurch gekennzeichnet, dass** die Inkubationsvorrichtung (1) einen ersten Verteiler (14) zur Verteilung des in der ersten Vermischungsanordnung (2) vermischten reaktiven Fluids (F) aus der ersten Vermischungsanordnung (2) in die Behälter (4) aufweist, vorzugsweise, dass jeder Behälter (4) über den ersten Verteiler (14) durch reaktives Fluid (F) eines jeden Mischers (5) und/oder Tangentialflussfilters (6) der ersten Vermischungsanordnung (2) befüllbar ist, und/oder, dass die Inkubationsvorrichtung (1) einen zweiten Verteiler (15) zur Verteilung des inkubierten reaktiven Fluids (F) aus den Behältern (4) aufweist, vorzugsweise, dass jeder Mischer (9) und/oder Tangentialflussfilter (10) der zweiten Vermischungsanordnung (7) über den Verteiler (15) durch reaktives Fluid (F) eines jeden Behälters (4) befüllbar oder durchströmbar ist, und/oder, dass der erste Verteiler (14) und/oder der zweite Verteiler (15) derart ausgebildet ist, dass wahlweise Behälter (4) mit dem ersten und/oder zweiten Verteiler (14, 15), insbesondere werkzeuglos, verbindbar und/oder davon trennbar sind.

15. Inkubationsvorrichtung nach einem der Ansprüche 9 bis 14, **dadurch gekennzeichnet, dass** die Behälter (4) hängbar, insbesondere nach Art einer hängbaren Tasche oder eines hängbaren Beutels, ausgebildet sind, vorzugsweise, dass die hängbar ausgebildeten Behälter (4) jeweils eine Öse (22) zum Hängen des Behälters (4) aufweisen, und/oder, dass die Behälter (4) jeweils zumindest eine Druckausgleichsöffnung (23) zum Druckausgleich in dem jeweiligen Behälter (4), insbesondere mit einem Öffnungsfilter (28), aufweisen.

16. Inkubationsvorrichtung nach einem der Ansprüche 9 bis 15, **dadurch gekennzeichnet, dass** die Inkubationsvorrichtung (1) ein Wiegemittel aufweist, vorzugsweise eine Wägezelle und/oder einen Kraftaufnehmer, und/oder, dass die Inkubationsvorrichtung (1) zumindest einen Durchflusssensor zur Regulierung der Füllmenge an reaktivem Fluid (F) in den Behältern (4) aufweist, und/oder, dass die Inkubationsvorrichtung (1) zumindest eine Füllstandsonde zum Erfassen des Füllstands an reaktivem Fluid (F) in den Behältern (4) aufweist, wobei über das Wiegemittel die jeweilige Masse des reaktiven Fluids (F) in dem Behälter (4) und über den Durchflusssensor der jeweilige Fluidstrom in den Behälter (4) und/oder aus dem Behälter (4) und über die Füllstandsonde der jeweilige Füllstand des reaktiven Fluids (F) in den Behältern (4) bestimmbar ist.

17. Inkubationsvorrichtung nach einem der Ansprüche 9 bis 16, **dadurch gekennzeichnet, dass** die Inkubationsvorrichtung (1) eine der ersten Vermischungsanordnung (2) in Strömungsrichtung nachgeschaltete Pumpe (24), insbesondere eine Peristaltikpumpe, zum Pumpen des in der ersten Vermischungsanordnung (2) vermischten reaktiven Fluids (F) zu den Behältern (4) aufweist, und/oder, dass die Inkubationsvorrichtung (1) eine den Behältern (4) in Strömungsrichtung nachgeschaltete Pumpe (25), insbesondere eine Peristaltikpumpe, zum Pumpen des inkubierten reaktiven Fluids (F) zu der zweiten Vermischungsanordnung (7) oder zu der Trennanordnung (8) aufweist.
